# EUROPEAN PATENT APPLICATION

(11) **EP 1 251 435 A2**
(43) Date of publication of application: **23.10.2002**
(21) Application number: 02003746.1
(22) Date of filing: 19.02.2002
(51) Int. Cl.: G06F 17/30, G06F 19/00

(54) **Knowledge database and method for constructing and merging knowledge database**

(30) Priority: 14.03.2001 JP 2001071769
(71) Applicant: Hitachi Software Engineering Co., Ltd., Yokohama-shi, Kanagawa 231-8475 (JP); Okubo, Kousaku, Minoo-shi, Osaka 562-0045 (JP)
(72) Inventor: Okubo, Kousaku, Minoo-shi, Osaka 562-0045 (JP); Tamura, Takuro, Naka-ku, Yokohama-shi, Kanagawa 231-8475 (JP); Yamashita, Iwao, Naka-ku, Yokohama-shi, Kanagawa 231-8475 (JP)
(74) Representative: Liesegang, Roland, Dr.-Ing.

(57) **Abstract**

Disclosed is a technology capable of acquiring transverse knowledge beyond specialized fields such as medicine and biology. From a knowledge database for accumulating objects as object values, the objects having phrases including terms used in a medical field and/or a biological field, and accumulating relations between the objects, the relations having values, as relation values, for expressing mutual relations between the objects quantitatively, objects related to a query object are extracted based on the relations between the objects, and the query object and the objects extracted are displayed together with the relations therebetween.

## Description

### BACKGROUND OF THE INVENTION

### Field of the Invention

The present invention relates to a technology for reconstructing knowledge accumulated in various forms in a medical field or a biological field as "substance names and events" (objects) constituting the knowledge and mutual relations therebetween and thus making it possible to structuralize the knowledge.

### Prior Art

Recent years, as medical/biological researches have flourished, a large amount of research results have been reported. The research results have been accumulated as texts such as academic treatises, textbooks and articles, and as data such as base sequences of DNA (DNA sequences), amino acid sequences of protein (protein sequences) and three-dimensional coordinates. The amount of such information has continued to be radically increased. The textbooks have come to be thicker, and with regard to the amount of reported DNA/protein sequences, it does not take one year to double an accumulation amount thereof under the present situation.

As an example of accumulating such a large amount of medical/biological information and realizing access to the information, enumerated is the PubMed database (http://www.ncbi.nlm.nih.gov/) operated by the National Center for Biotechnology Information (NCBI) of the National Library of Medicine (NLM). In the PubMed, an enormous amount of information concerning academic treatises related to the medicine/biology is converted into a database. The above-described information concerning the academic treatises can be accessed by use of phrases included in the title of the abstract, author names, years issued, related DNA/protein sequence information, related treatises and the like, which are includes in titles and abstracts.

Moreover, as another example, there are GeneCards (http://bioinformatics.weizmann.ac.il/cards/) by Michael Rebhan et.al., in the Weizmann Institute of Science. In the GeneCards, a system is prepared, in which information related to genes is automatically collected from public databases on the Internet and the information is integrated for individual genes as units. Thus, providing of up-to-date and exhaustive information related to target genes is realized.

### SUMMARY OF THE INVENTION

In many academic fields, it is conceived that development of researches leads to discovery of the truth, a large amount of knowledge connected to hypotheses proved to be incorrect is abolished at that point of time, whereby information is ordered, and based on the ordered information, the next research is going to be developed. However, in the medical/biological fields, it is conceived that fragmented knowledge is being accumulated and information is being multiplied without ordering information in the above-described manner. Moreover, many researchers have delved into specialized fields of their own. Consequently, the researches have been specialized and ramified, thus making it difficult to obtain the truth from transverse information.

In the prior art, information has been managed for each knowledge unit such as the academic treatises, the books and the Web page. In order to obtain information beyond the knowledge units, for example, in order to obtain knowledge derived logically from a plurality of knowledge units, it has been necessary to acquire knowledge units required therefor, to understand and memorize the contents thereof and to connect the contents in mind.

The above-described one means that structures and relations between knowledge elements constituting the knowledge units (hierarchical structures and relational structures) have not been able to be obtained or utilized as information though the knowledge units have been able to be accumulated. For the same reason, it has not been possible to derive the knowledge logically from different types of information such as the knowledge in the medical/biological fields, which is expressed by natural languages (lingualized knowledge), and the DNA/protein sequence information.

A system for handling the relations between the knowledge elements is realized and applied to the medical/biological fields. Thus, knowledge beyond specialized fields such as anatomy, clinic and biochemistry is coupled one another; therefore, it is made possible to observe manifold knowledge and to uncover potential knowledge. Moreover, it is made possible to provide means for deriving necessary information from a large amount of information generated in the medical/biological researches. Furthermore, it is made possible to assist understanding of the knowledge contents by graphical representation thereof, to discover another expression for explaining certain knowledge, to discover a hierarchical structure of the knowledge and to detect the same knowledge by different ways of expression. The present invention has the object to provide means for enabling the above-described matters.

In the present invention, in order to achieve the foregoing object, target knowledge is defined by an "object" as a knowledge element having one value corresponding to a knowledge type and by a "relation" as a relation between objects, which has one nondirectional value, thus it is made possible to carry out information processing such as merge, extraction and illustration of the knowledge, and knowledge information processing is realized, to which the above information processing is applied.

Concretely, as types of objects, defined are: "phrase objects" having phrases such as technical terms, idioms and clauses constituting the knowledge as knowledge elements, with knowledge expressed by a natural language, particularly, knowledge (lingualized knowledge) in the medical/biological fields taken as a target; "DNA sequence objects having DNA sequences as knowledge elements and "protein sequence objects" having protein sequences as knowledge elements, with the DNA sequences and the protein sequences taken as targets; and "image objects" having images as knowledge elements. For example, in the phrase objects, a relation value between the objects is set based on a frequency of existence of two phrases being close to each other in the lingualized knowledge as a knowledge resource. Moreover, in the DNA sequence objects and the protein sequence objects, the relation value between the objects is set based on a homology score between the DNA sequences or between the protein sequences (Smith, T. F. & Waterman, M. F. 1990, Proc. Nat. Acad. Sci., U.S.A. 87, 1118-122) or a value obtained by the maximum coincidence length therebetween and a coincidence rate thereof. Furthermore, in the image objects, the relation value between the objects is set based on a score of pattern matching of the images, a similarity score between characteristic values (luminance, particle size and the like) of the image or a value set based on conditions of acquiring the image.

Here, when one piece of knowledge information composed of relations between a plurality of objects and the entire objects is set as a "knowledge database", the objects are defined to have unique values in the knowledge database. Thus, when two knowledge databases are merged, it is made possible to fuse two knowledge aggregates via objects having the same value. For example, two knowledge databases created from information resources (knowledge resources) in different specialized fields are merged, thus making it possible to generate a knowledge database obtained by fusing the knowledge of two specialized fields.

Definition of a calculation system for the relation values is designed to be capable of being set according to the purpose of using the knowledge database. For example, the relations between the DNA sequence objects or the protein sequence objects can be set based on homologies of the DNA sequences or the protein sequences to be used as information concerning physical similarities. Alternatively, the relations can be set based on results of measurement experiments for gene occurrence profiles to be used as information concerning molecular-biological property.

The relation values are designed to be capable of expressing "relation sizes" and "that two target objects are regarded as the same". Thus, expression is enabled for regarding a certain set of objects as the same in different types of objects.

Here, description will be made below for means for achieving the foregoing object according to the present invention.
(1) A knowledge database comprising: a plurality of phrase objects having phrases included in natural language information as object values; and relations between the phrase objects, the relations having values expressing the relations between the objects quantitatively as relation values.

The phrases include terms, idioms, clauses and the like.
(2) A knowledge database comprising: a plurality of objects having any of DNA sequences and protein sequences as object values; and relations between the objects, the relations having values obtained by any of homology calculation and experimental measurement for any of relations between the DNA sequences and relations between the protein sequences to be expressed quantitatively as relation values.
(3) A knowledge database comprising: a plurality of objects; and relations between the objects, wherein the objects include any of DNA sequence objects having DNA sequences as object values and protein sequence objects having protein sequences as object values, and phrase objects having phrases included in natural language information related to any of the DNA sequences and the protein sequences as object values, and any of relations between the DNA sequence objects and the phrase objects and relations between the protein sequence objects and the phrase objects have relation values previously defined.

The phrases included in the natural language information related to the DNA sequences or the protein sequences also include identifiers (accessing numbers, names, marks, codes, identification numbers and the like) for identifying the DNA sequences or the protein sequences.

Whichever certain two objects may be the same type or different types, definition of the calculation system for the relation values is designed to be capable of being set according to the types of the two objects. Thus, it is made possible to express the knowledge by combining different types of objects. Moreover, also for objects of the same type, it is set possible to define the calculation system for the relation values in accordance with the purpose, thus making it possible to express the knowledge so as to meet the purpose. In other words, even if the objects are the same, different knowledge databases are constructed by changing the way of defining the relations.
(4) A knowledge database comprising: a plurality of objects having images as object values; and relations between the objects, the relations having values expressing the relations between the objects quantitatively as relation values.
(5) A knowledge database comprising: a plurality of objects; and relations between the objects, wherein the objects include image objects having images as object values and phrase objects having phrases included in natural language information related to the images as object values, and relations between the image objects and the phrase objects have relation values previously defined.

The phrases included in the natural language information related to the images also include identifiers (marks, codes, identification numbers, file names, URLs and the like) for identifying the images.
(6) A knowledge database comprising: a plurality of objects; and relations between the objects, wherein the objects include phrase objects having phrases including terms for use in a medical field and/or a biological field as object values, and the relations have values expressing the relations between the phrase objects quantitatively as relation values.
(7) The knowledge database according to (6), wherein a synonym object dictionary is provided, the synonym object dictionary registering a plurality of objects to be regarded as a same object therewith as a synonym object group.
(8) The knowledge database according to (6), wherein the objects include any of DNA sequence objects having DNA sequences as object values, protein sequence objects having protein sequences as object values and image objects having images as object values, and any of relations between the DNA sequence objects and the phrase objects, relations between the protein sequence objects and the phrase objects, and relations between the image objects and the phrase objects have relation values previously defined.
(9) The knowledge database according to (8), wherein a synonym object dictionary is provided, the synonym object dictionary registering any of a plurality of DNA sequence objects having highly homologous DNA sequences as object values and protein sequence objects having highly homologous protein sequences as object values therewith as a same object group.

As a method for avoiding dilution of information caused by a linguistic sway, a difference in expression due to a difference in specialized field, an error of information in an experiment and the like in the objects obtained from the knowledge resource, the knowledge database of the present invention prepares a method for regarding a plurality of objects as objects equivalent from one to another (synonym object group) and for merging relations related thereto. The entire objects included in the synonym object group are related to the knowledge database to be saved as synonym object information. Moreover, in the knowledge database, an object representing the synonym object group (object representative of synonyms) is set to be registered therewith.

In updating the knowledge database in which the synonym object group has already been set, in the case where objects to be updated exist in the synonym object information, a relation of the object representative of synonyms in the target synonym object group is updated.

When a synonym object group is newly set for the knowledge database, the object representative of synonyms is generated to be registered with the knowledge database, and the objects included in the synonym object group are deleted from the knowledge database to be saved as synonym object information. The relations that have been related to the objects included in the synonym object group are entirely merged to be set as relations between the object representative of synonyms and the other objects.

For the phrase objects, a plurality of designated phrase objects are registered as a synonym object group. Values of the phrase objects included in the synonym object group are set as values caused by a linguistic sway, a difference in expression due to a difference in specialized field, frequent erroneous description and the like. As the object representative of synonyms, an optional phrase object is selected.

In the DNA sequence objects or the protein sequence objects, for example, in a range of a designated sequence of more of the DNA sequences or the protein sequences, the DNA sequence objects or the protein sequence objects, which have a homology of a certain value or higher, are registered as a synonym object group. As the object representative of synonyms, an optional DNA sequence object or an optional protein sequence object is selected. Alternatively, a consensus sequence is created and registered.

A synonym object dictionary registering therewith a plurality of synonym object groups is prepared, thus making it possible to set an equivalent synonym object group for different knowledge databases.

In an operation of knowledge databases, which is to be described later, the synonym object information is merged and reflected on the knowledge database prior to an operation of the objects and the relations. Thus, it is made possible to avoid mismatch between the objects and the synonym object information in the knowledge database after the operation. Moreover, for an effective operation, it is effective to previously make synonym object groups common by use of the synonym object dictionary.
(10) The knowledge database according to (1) or (6), wherein the phrase objects have phrases in knowledge expressed by a natural language (lingualized knowledge) as object values, and have values obtained by quantifying frequencies of existence of the respective phrases being close to each other in the lingualized knowledge as relation values of relations between the corresponding phrase objects.

The phrases in the lingualized knowledge may be generated by decomposing the lingualized knowledge into sentences by previously prepared sentence-separating letter strings, each being composed of one letter or a plurality of letters, then decomposing the sentences into phrases by previously prepared phrase-separating letter strings, each being composed of one letter of a plurality of letters. Alternatively, the phrases in the lingualized knowledge may be the one designated optionally by a user. Moreover, that the phrases exist close to each other in the lingualized knowledge signifies, for example, that the phrases exist in the same page, the same paragraph or the same sentence of a document.
(11) The knowledge database according to (1) or (6), wherein the phrase objects have phrases included in an index of a book as object values, and have a value obtained by quantifying a frequency of existence of two phrases being on any of a same page and a same paragraph of the book as a relation value of a relation between the corresponding two phrase objects.
(12) The knowledge database according to (1) or (6), wherein the relations between the phrase objects have, as relation values, values obtained by quantifying similarities of patterns (existing profiles) in existence of the phrases in knowledge units expressed by a natural language.

The knowledge unit means a page or a clause of a book or the like, a Web page, a DNA database entry and the like. Cluster analysis is performed for the knowledge unit based on the occurrence pattern of the phrase objects and the occurrence frequency pattern in the knowledge unit, and similarity distances between the phrases are obtained to be set as relation values.
(13) A method for displaying objects and relations, comprising the steps of: extracting objects related to an object corresponding to one or a plurality of keywords from a knowledge database based on relations between the objects, the object corresponding to the keywords being a query object, and the knowledge database including the objects having phrases including terms for use in a medical field and/or a biological field as object values and the relations between the objects, the relations having values expressing the relations between the objects quantitatively as relation values; and displaying the query object and the extracted objects together with the relations therebetween.

The knowledge database may include a molecular structure such as the DNA sequences, the protein sequences and a protein higher order structure as a value of the object. Furthermore, the knowledge database may include an image such as a microscope picture of a lesion tissue as a value of the object.
(14) The method according to claim 13, wherein the query object is selected by inputting or searching the object values, and the selected query object, the extracted objects and the relations therebetween are displayed on a list or displayed graphically.
(15) The method according to claim 13, wherein objects having strong relations with the query object are obtained hierarchically, and the query object, the hierarchically obtained objects and relations therebetween are displayed on a list or displayed graphically.

The objects having strong relations are objects having large relation values of the relations between the objects. A degree of the relation values as strong relations may be designated optionally by the user. Alternatively, the number of relations to be employed from the one having a larger relation value may be set so that the number of objects obtained from the query object can be a certain value or less. Preferably, in the objects or the relations displayed on a list or displayed graphically, one or a plurality of objects or relations can be selected, and the selected one or plurality of objects should be newly set as query objects, thus obtaining a target object. Moreover, the selected plurality of objects can be registered in the synonym dictionary as synonyms.
(16) A method for constructing a knowledge database, comprising the steps of: extracting phrases used in expression of knowledge of a medical field and/or a biological field from the knowledge expressed by a natural language (lingualized knowledge); generating objects having the phrases as object values; generating relations between the objects, the relations having values expressing the relations between the objects quantitatively as relations values; and accumulating the generated objects and the relations between the objects.
(17) The method for constructing a knowledge database according to (16), wherein the lingualized knowledge is decomposed into sentences by previously prepared sentence-separating letter strings, each being composed of one letter or a plurality of letters, the sentences are decomposed into phrases by previously prepared phrase-separating letter strings, each being composed of one letter or a plurality of letters, and the phrases obtained are set as the object values, and a frequency of existence of two phrases being close to each other in the lingualized knowledge is set as a relation value of a relation between the corresponding two phrase objects.
(18) The method for constructing a knowledge database according to (16) or (17), wherein an index of a book, a table of contents of a book, a title of an academic treatise, a body text of a book, a body text of an academic treatise and/or a body text of a Web page are used as the lingualized knowledge.
(19) The method for constructing a knowledge database according to (16), wherein phrases included in the index of the book are used as master data of phrase objects, and a frequency of existence of the two phrases being on a same page of the book is set as a relation value of a relation between the corresponding two objects.

As the book, a textbook, a handbook, a manual, a dictionary and the like can be used.
(20) The method for constructing a knowledge database according to (16), wherein previously designated optional phrases are used as master data of phrase objects, and a frequency of existence of the two phrases being closer to each other in the lingualized knowledge is set as a relation value of a relation between the corresponding two objects.
(21) The method for constructing a knowledge database according to (16), wherein academic treatises are used as the lingualized knowledge, information including titles and/or body texts of the academic treatises is periodically acquired through a network, phrase objects and relations are extracted from the acquired information, and the knowledge database is updated.
(22) The method for constructing a knowledge database according to (16), wherein a plurality of objects to be regarded as a same object and relations related thereto are merged.

The objects to be regarded as the same object can be designated by the user.
(23) A method for constructing a knowledge database, comprising the steps of: extracting DNA sequences from information including DNA sequences; generating DNA sequence objects having the DNA sequence as object values; generating a relation between two DNA sequence objects, the relation having, as a relation value, a quantitative value obtained by any of homology calculation and experimental measurement for a relation between the corresponding two DNA sequence; and accumulating the generated DNA sequence objects and the generated relation between the DNA sequence objects.
(24) The method for constructing a knowledge database according to (23), wherein phrases included in natural language information related to the DNA sequences are extracted, objects having the extracted phrases as the object values are generated, and relations having defined relation values with the DNA sequence objects corresponding to phrase objects are set.

In addition to the DNA sequences, the protein sequences may be converted into objects similarly. As the lingualized knowledge used for constructing the knowledge database by extracting the objects and the relations, lingualized knowledge including the DNA sequences, the protein sequences, both of them or information added thereto can be used. And, a database accumulating the DNA sequences, the protein sequences, both of them or information added thereto can be used. Moreover, knowledge expressed by structured descriptive expression such as an HTML and an XML can be also used.
(25) The method for constructing a knowledge database according to (24), wherein information including the DNA sequences and natural language information related thereto is periodically acquired through a network, the DNA sequence objects, the phrase objects and the relations are extracted from the acquired information, and the knowledge database is updated.
(26) The method for constructing a knowledge database according to (23), wherein a plurality of the DNA sequence objects having highly homologous DNA sequences as object values are regarded as a same object, and the plurality of DNA sequence objects regarded as the same object and relations related thereto are merged.
(27) A method for generating a knowledge database, comprising the steps of: performing an operation between first and second databases, each database accumulating a plurality of objects having, as object values, phrases expressed by a natural language, and relation values between the plurality of objects, the relations having, as relation values, values expressing relations between the objects quantitatively; and generating a third knowledge database.

As the operation between the knowledge databases, enumerated are addition, subtraction and the like.
(28) A method for displaying a graph, comprising the step of: displaying a graph for comparing differences between relation values of focused relations in the first and second knowledge databases by use of the first and second databases, each database accumulating a plurality of objects having, as object values, phrases expressed by a natural language, and relation values between the plurality of objects, the relations having, as relation values, values expressing relations between the objects quantitatively.

When the above method is executed for a plurality of knowledge databases generated from a series of knowledge resources having significance in time series, there can be displayed a graph for comparison of changes of the knowledge included in the knowledge resources in time series.
(29) The method according to (28) or (29), wherein the relation values of the relations between the phrase objects included in each knowledge database are standardized between the plurality of knowledge databases.

As the standardization method, there are a standardization method based on an occurrence frequency of the objects in the knowledge resource as source data of the knowledge database, a standardization method in which values of focused relations in the knowledge database are equalized and the like. In the operation between the knowledge databases or in the comparison for the knowledge databases, it is desirable to prevent the mismatch between the objects and the synonym information in the target knowledge database after the operation or during the comparison by previously integrating the synonym object groups in the knowledge database.

According to the present invention, the lingualized knowledge such as an academic treatise and the encoded knowledge such as the DNA sequences and the protein sequences are reconstructed as a structure of a knowledge frame, which can be subjected to information processing by the objects such as phrases, events, marks and the relations therebetween, and knowledge related to certain knowledge is acquired by use of the structure of the knowledge frame, thus enabling explanation of knowledge, expression of knowledge and discovery of knowledge.

### BRIEF DESCRIPTION OF THE DRAWINGS

Fig. 1 is a view of a system configuration of the present invention.
Fig. 2 is a conceptual view of a simplest knowledge database.
Fig. 3 is a conceptual view of a knowledge database.
Fig. 4 is a conceptual view of merge of knowledge databases.
Fig. 5 is a conceptual view of merge of objects and relations in a knowledge database.
Fig. 6 is a view showing an example of a knowledge database including different types of objects.
Fig. 7 is an explanatory view of extraction of object/relation by a relation value.
Fig. 8 is a flowchart showing one example of general cutting-out of a phrase object and a procedure of relation setting.
Fig. 9 is an explanatory view showing an example of the cutting-out of the phrase object and the relation setting.
Fig. 10 is a flowchart showing one example of cutting-out of a phrase object from an index and a procedure of relation setting.
Fig. 11 is an explanatory view showing an example of the cutting-out of the phrase object from the index and the relation setting.
Fig. 12 is a view showing an example of generation of a knowledge database including a DNA sequence object from a DNA database entry.
Fig. 13 is a view showing an example of generation of a knowledge database including an image object from an image file and explanation of the image.
Fig. 14 is a view showing an example of generation of a knowledge database from a Web page.
Fig. 15 is a view showing an example of a knowledge database structure.
Fig. 16 is a view showing an example of object values and relation values in a knowledge database.
Fig. 17 is a view showing an example of data storage in the knowledge database structure.
Fig. 18 is a view showing an example of comparative graphs for knowledge databases.
Fig. 19 is a view showing an example of a display for objects and relations included in a knowledge database.
Fig. 20 is a view showing an example of a hierarchical display (circular display) of related objects.
Fig. 21 is a view showing an example of a hierarchical display (parallel display) of the related objects.
Fig. 22 is a view showing an example of search for knowledge database information from cluster analysis.

### DETAILED DESCRIPTION OF THE PREFERRED EMBODIMENT

Hereinafter, description will be made for an embodiment of the present invention with reference to the drawings.

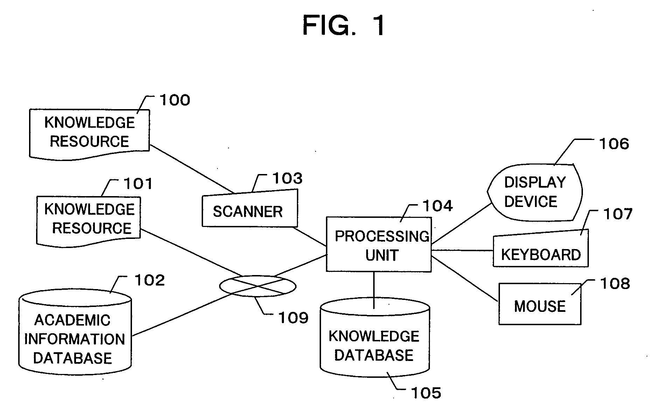

Fig. 1 is a view of a system configuration of the present invention. The system of the present invention is composed of the printed lingualized knowledge data 100 of medicine and biology, such as a textbook and a handbook, the lingualized knowledge data 101 such as an academic treatise and knowledge information, which is laid open on a remote system accessible through the network 109 and sequentially updated, the academic information database 102 such as DNA sequence information, protein sequence information and lingualized knowledge related thereto, which is laid open on a remote system accessible through the network 109 and sequentially updated, the scanner 103 for reading the printed lingualized knowledge as an image, the processing unit 104 for converting the image obtained through the scanner into text, and constructing a knowledge database taking, as resources, not only the converted lingualized knowledge (text) but also the lingualized knowledge, the DNA sequence and the protein sequence, which are obtained through the network, and thus for utilizing the knowledge database constructed, the database 105 for accumulating knowledge databases constructed in the processing unit 104, the display device 106 for visualizing a relation between knowledge elements of medicine and biology knowledge and displaying the relation, and the keyboard 107 and the mouse 108 for performing value input and selection operations for this system.

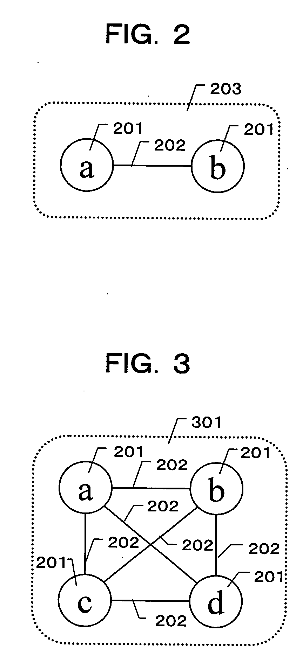

Fig. 2 is a conceptual view of the simplest knowledge database. Specifically, the knowledge database 203 of the present invention is composed of the objects 201 and the relation 202 therebetween. The relation is a nondirectional relation value (a scholar value) between two objects defined in the knowledge database. Moreover, as shown in a conceptual view of the knowledge database of Fig. 3, it is assumed that the relations 202 exist between all of the objects 201 existing in the knowledge database 301.

By merging the two knowledge databases (by obtaining the sum of the knowledge databases), it is made possible to increase an information amount of the knowledge database and to generate a knowledge database including potential knowledge existing in different specialized fields. Moreover, by obtaining a difference between the two knowledge databases, a knowledge database including unique knowledge and a bias of knowledge can be generated between the knowledge databases. Such processing between the knowledge databases (an operation of the knowledge databases) can be realized by generating a knowledge database having the entire objects owned by both the knowledge databases as operation sources, and moreover having relation values calculated based on a relation originating from both the knowledge databases as the operation sources or relation values calculated from object values. In the calculation of the relation values in the operation of the knowledge databases, with regard to a relation not having existed in the knowledge databases as the operation sources, a value of the relation is set as zero (0), and then the calculation is carried out. Moreover, with regard to a relation having a value for "regarding two target objects as the same" in the knowledge databases as the operation sources, optionally, this value is maintained as a calculation result of the relation, alternatively this value is converted into a difference value, and then the calculation for the relation is carried out.

In the sum of the knowledge databases, the calculation of the relation values may be carried out from the sum of the relation values in the two knowledge databases as the operation sources.

In the difference between the knowledge databases, the calculation of the relation values may be carried out from the difference between the relation values in the two knowledge databases as the operation sources. In the calculation, when a relation value on the left side of the operation is smaller than a relation value on the right side thereof, zero may be obtained as a calculation result of the relation value.

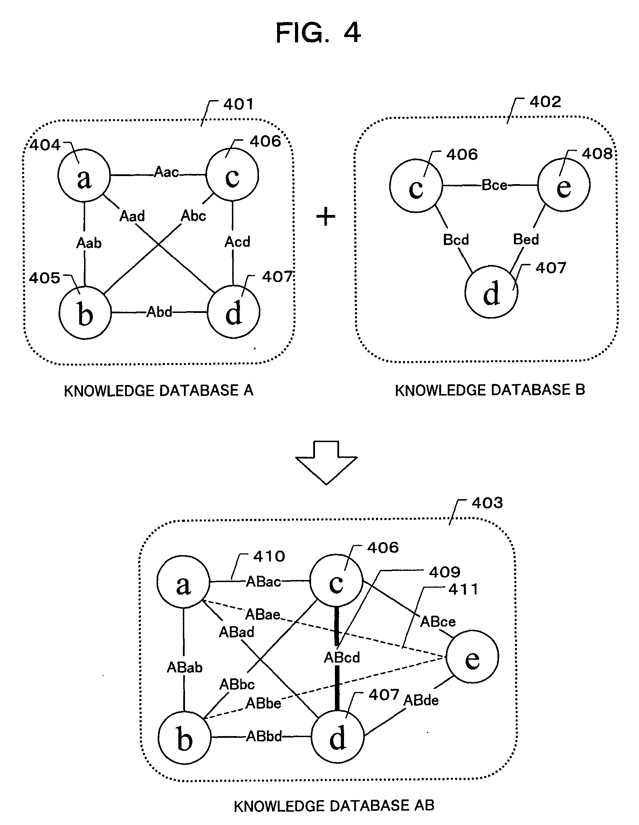

Fig. 4 is a conceptual view of merge of knowledge databases. When two knowledge databases are merged, objects in both the knowledge databases to be merged are compared with each other, and objects regarded as the same are merged. In this event, a relation between the two objects to be merged is deleted, and relation values between the two objects to be merged and all the other objects are calculated by a function or a recursion formula, which is designated depending on a type of the objects to be merged.

For example, it is assumed that the relation between the objects a and b in the knowledge database A is expressed as Aab, and that a function for calculating the relation between the objects a and b in the knowledge database AB to be generated by the merge of the knowledge database A and the knowledge database B is: ABab = f(Aab, Bab). Then, in the database AB (403) to be generated by the merge of the knowledge database A (401) and the knowledge data base B (402), the ABcd(409) as a relation between the objects c and d (406) and (407) having existed commonly in both the knowledge databases to be merged is calculated as: ABcd = f(Acd, Bcd). Moreover, a relation between the objects, which has existed in only one of the knowledge databases to be merged, for example, ABac (410) is calculated as: ABac = f(Aac, 0). Furthermore, ABae(411) as a relation newly generated by the merge is calculated as: ABae = f(0, 0). Values calculated as described above can be set as the relation values.

In this case, for example, a relation between a phrase object and other type of object calculates a value by a function: f(Aab, Bab) = Aab+Bab or maintains the relation value of any one of the objects. Moreover, in relations between the DNA sequence objects or between the protein sequence objects, the relation value is re-calculated based on the value of the merged object, or the relation value originating from any one of the knowledge databases is maintained. Furthermore, in the relation between the image objects, the relation value is re-calculated based on the value of the merged object, or the relation value originating from any one of the knowledge databases is maintained. In the merge of the knowledge databases, according to a method for judging objects as the same, for example, objects having phrases coincident with each other are regarded as the same in phrase objects. Moreover, in the DNA sequence objects or the protein sequence objects, for example, a homology calculation is performed, and a homology score, or a length of a coincidence range or a coincidence ratio in apposition of the maximum coincidence is set as a threshold value, and a set of the objects exceeding the threshold value is regarded as the same. Moreover, in the image object, for example, a pattern matching is carried out, and a set of the image objects exceeding a value set as the threshold value is regarded as the same. The value of the merged object is not changed in the phrase object, and moreover, in the DNA sequence object and the protein sequence object, for example, any one of the values of the objects may be set as a value of the merged object, alternatively, a consensus sequence may be created from values of the two objects in the knowledge databases to be merged to be set as a new value. Furthermore, in the image objects, for example, any one of the values thereof may be set as a value of the merged object.

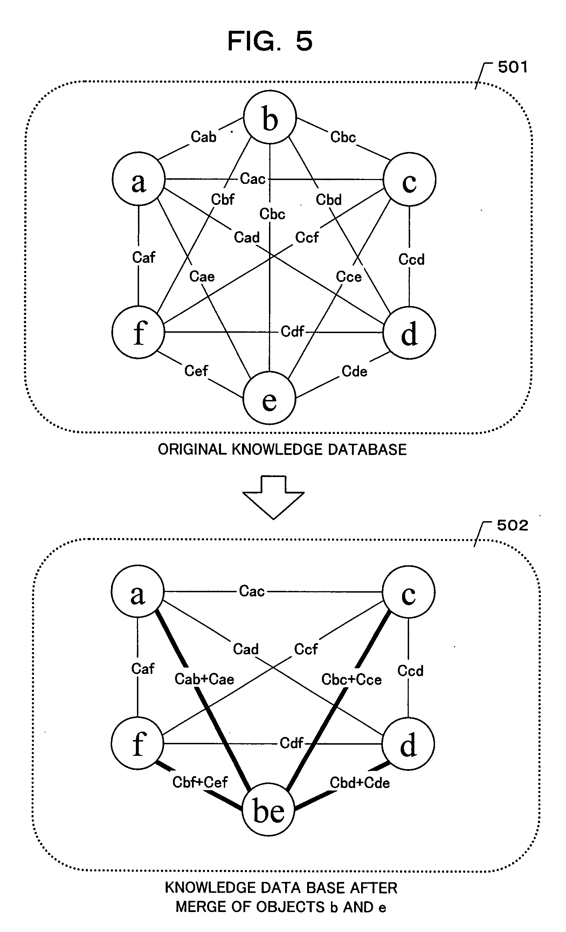

Fig. 5 is a conceptual view of merge of objects in a knowledge database. When two objects in the knowledge database are merged, the relation between both the objects to be merged is deleted, and the relations having existed between both the objects and all the other objects respectively are merged, then re-calculation is carried out.

The relation between the objects a and b in the knowledge database C is expressed as Cab, and moreover, a function for calculating the relation between the object c and the object ab generated by the merge of the object a and the object b is set as: Cab, c = f(Cac, Cbc). Then, Cbe, c as the relation between the object be and the object c, which exist in the merged knowledge database C (502), are calculated as: Cbe, c = f(Cbc, Cce) and set as a relation value. In this case, Cbe, c is generated as a result obtained by merging the objects b and e of the knowledge database C (501) not merged yet in Fig. 5. For example, with regard to the relation between the phrase object and other type of object, a value is calculated by: f(Cbc, Cce) = Cbc + Cce. Furthermore, for example, in the relations between the DNA sequence objects and between the protein sequence objects, the relation value is re-calculated based on the value of the merged object. The value of the merged object is not changed in the phrase object. Moreover, in the DNA sequence object and the protein sequence object, for example, any one of the values of the objects may be set as a value m, alternatively, a consensus sequence may be created from values of the two objects in the knowledge databases to be merged to be set as a new value.

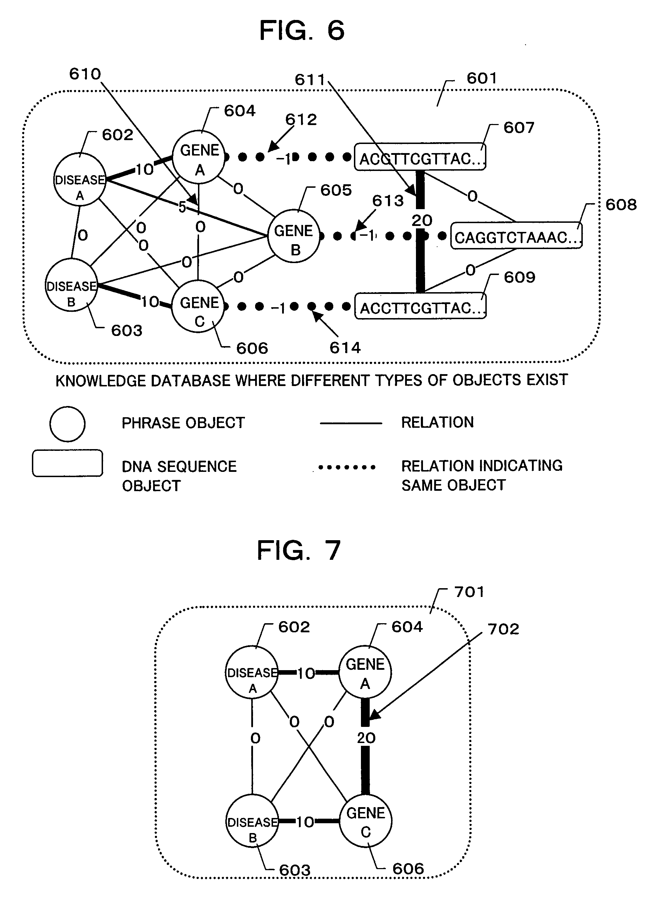

Fig. 6 is a view showing an example of a knowledge database including different types of objects. Here, with regard to relation values, when two target objects are regarded as the same, -1 is set, when no relation exists between the objects, 0 is set, and when a relation exists between the objects, a value larger than 0 is set. Specifically, the large the value is, the closer the relation between the objects is. In order to make the drawing easy to see, with regard to the relations between the objects different in type, illustration for the one having a value of 0 is omitted. For example, the relation between the phrase object 602 and the DNA sequence object 607 is omitted.

The knowledge database 601 includes the phrase objects 602 to 606 and the DNA sequence objects 607 to 609, and has relations not only between the objects of the respective types but also between different types of objects. For example, the following is shown in the knowledge database 601. With regard to the relation 612 between the objects 604 and 607, the relation 613 between the objects 605 and 608 and the relation 614 between the objects 606 and 609, all the values thereof are equal to -1, and these objects are regarded as the same.

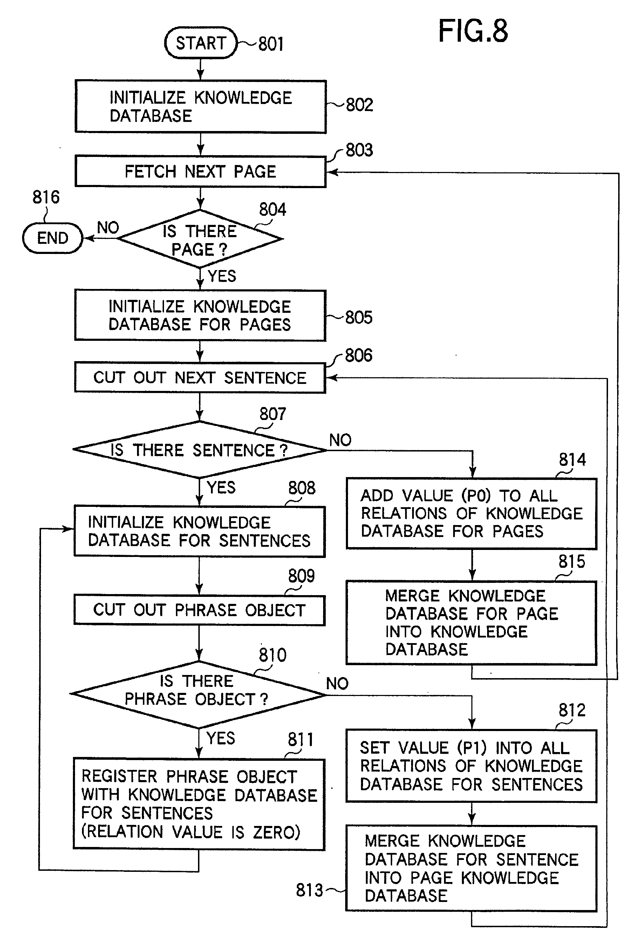

Fig. 7 shows an example of extracting the knowledge database 701 including the phrase objects related by relations having relation values of 10 or more from the knowledge database shown in the example of Fig. 6. Evaluation will be made only for the phrase objects in Fig. 6. The value of the relation 610 between the objects 604 and 606 is zero, which indicates that there is no relation therebetween. However, the relation 610 between the objects 604 and 606 can be merged into the relation 611 between the objects 607 and 609 defined to be regarded as the same by the relations 612 and 614 respectively, and thus the relation 702 having a value of 20 can be obtained. As described above, it is possible to obtain knowledge information through different types of objects based on the relation values from the constructed knowledge database.

Extraction of phrase objects from a knowledge resource as lingualized knowledge can be performed by, for example, a method, which comprises the steps of decomposing the lingualized knowledge into sentences by previously prepared sentence-separating letter strings, each being composed of one letter or a plurality of letters, decomposing the sentences into phrases by a previously prepared phrase-separating letter strings, each being composed of one letter or a plurality of letters, and defining the phrases obtained as phrase objects. In this event, the relation values may be set depending on frequencies where the same phrases exist in the same page or sentence.

Fig. 8 is a flowchart showing one example of a procedure for cutting out phrase objects from a knowledge resource and setting relations therebetween in the case where lingualized knowledge is set as the knowledge resource.

The processing is started (801), then a knowledge database to be created is prepared and initialized (802). At this point of time, in the knowledge database, objects do not exist, and accordingly, relations do not exist either. Subsequently, a page is fetched as a data unit from the knowledge resource (803). In this example, as data units, pages and sentences are taken. When two objects exist in the same page, a score of a relation therebetween is set as P0. When two objects exist in the same sentence, a score of a relation therebetween is set as P1. Then, decision is made as to whether or not a page exists (804). When a page exists, processing for adding information of the page to the knowledge database to be created is carried out. As a procedure thereof, first, a knowledge database for accumulating page knowledge (a knowledge database for pages) is prepared and initialized (805).

Subsequently, sentences are cut out from each page (806). For cutting out the sentences, previously registered "sentence-cutting-out letter strings", each being composed of one letter or a plurality of letters, is used. For example, ". (period)" and "(line feed)" are registered with the sentence-cutting-out letter strings. Lingualized knowledge included in the page is split into sentences by the sentence-cutting-out letter strings. Decision is made as to whether or not a sentence exists (807). When a sentence exists, a knowledge database for accumulating sentence knowledge (a knowledge database for sentences) is prepared and initialized (808). Subsequently, phrases are cut out from each sentence to be generated as phrase objects (809). For cutting out the phrases, previously registered "phrase-cutting-out letter strings", each being composed of one letter or a plurality of letters (809), is used. For example, "and", "that" and "(Tab)" are registered with the phrase-cutting-out letter strings. Lingualized knowledge included in the sentence is split by the phrase-cutting-out letter strings, and spaces are deleted from a head and an end of each letter string obtained by the split, thus each letter string is set as a phrase. Decision is made as to whether or not a phrase object exists (810). When a phrase object exists, the phrase object is registered with the knowledge database for sentences (811), and then, the next object is cut out. In registering the phrase object with the knowledge database for sentences, the relation value is set as zero. When a phrase object does not exist, judgment is made that the extraction of the objects from a target sentence has been entirely completed, and the value P1 is added to the entire relations of the knowledge database for sentences (812), then the knowledge database for sentences are merged into the knowledge database for pages (813). Thereafter, the next sentence is cut out.

Decision is made at to whether or not a sentence exists (807). When a sentence does not exist, judgment is made that the extraction of the objects from a target page has been entirely completed, and the value P0 is added to the entire relations of the knowledge database for pages (814), then the knowledge database for pages is merged into the knowledge database to be created (815). Thereafter, the next page is fetched. Decision is made as to whether or not a page exists (804). When a page does not exist, the processing is terminated, and creation of the knowledge database to be created is completed (816). By the above procedure, the phrase objects can be cut out from the lingualized knowledge and the relations can be set.

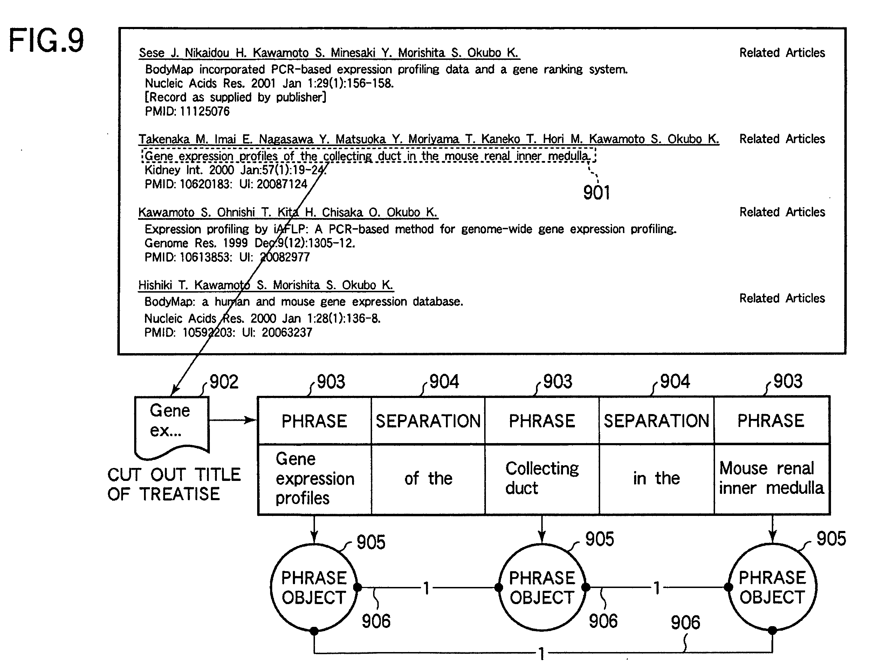

Fig. 9 is a view showing a case where a title of a treatise is set as a resource of knowledge, the case being as another example of the method for cutting out phrase objects from lingualized knowledge and for setting relations therebetween. The title 901 of a treatise is available by browsing and searching information through the Internet or from information distributing services or books. Such a treatise title is cut out (902), the title is split into the phrases 903 by the phrase-cutting-out letter strings 904, and each phrase is set as the object 905, then the relations 906 between the phrases are set. Here, the relation value 1 (one) is set.

As another method for extracting phrase objects from a knowledge resource as lingualized knowledge, for example, there is a method, in which phrases (e.g., terms, idioms and clauses) appearing in an index of a book such as a textbook, a handbook, a manual and a dictionary are previously collected, and the phrases are used as master data of the phrase objects, then the same phrases are extracted from a target knowledge resource. In this event, as for setting of relations, for example, values thereof may be set depending on frequencies where the same phrases exist in the same page, based on page numbers extracted from the index simultaneously with the phrases.

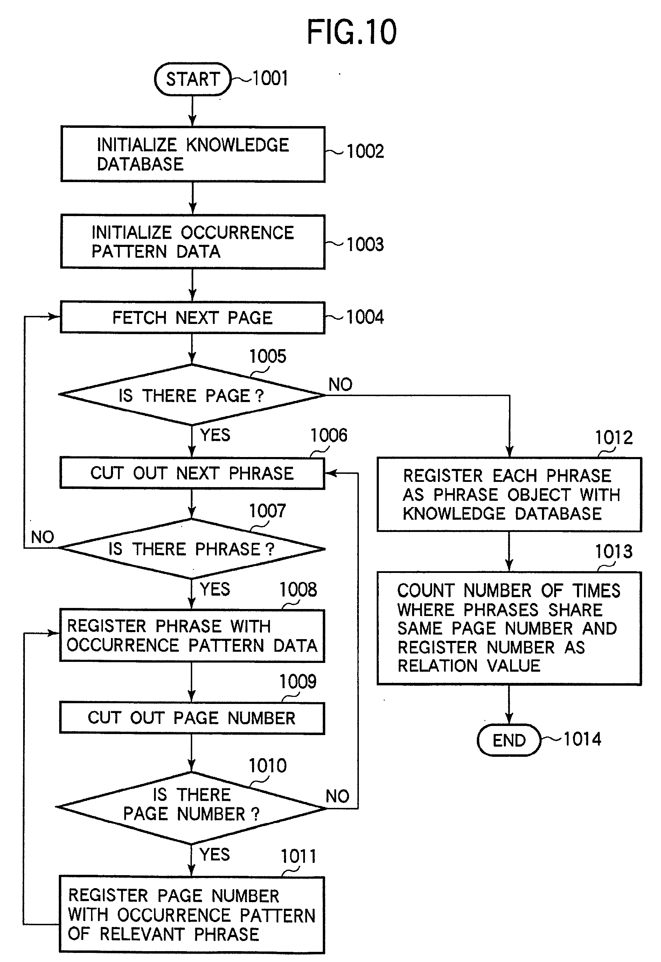

Fig. 10 is a flowchart showing one example of a procedure for cutting out phrase objects from a knowledge resource and setting relations therebetween in the case where a book index is set as the knowledge resource.

The processing is started (1001), then a knowledge database to be created is prepared and initialized (1002). At this point of time, in the knowledge database, objects do not exist, and accordingly, relations do not exist either. Subsequently, occurrence pattern data is initialized (1003). In the occurrence pattern data, stored is as to which page number is owned by each phrase included in the index. Next, a page including the index is fetched from the knowledge resource (1004). Decision is made as to whether or not a page exists (1005). When a page exists, a phrase included in the index is cut out (1006). Decision is made as to whether or not a phrase exists (1007). When a phrase exists, the phrase is registered with the occurrence pattern data (1008). A page number related to the phrase is cut out (1009). Decision is made as to whether or not a page number exists (1010). When a page number exists, the page number is registered with the occurrence pattern data (1011), and then the next page number is cut out (1009). Decision is made as to whether or not a page number exists (1010). When a page number does not exist, the next phrase is cut out (1006).

Decision is made as to whether or not a phrase exists (1007). When a phrase does not exist, the next page is fetched (1004). Decision is made as to whether or not a page exists (1005). When a page does not exist, judgment is made that reading-out of the entire indexes has been completed, and each object is registered as a phrase object with the knowledge database (1012). Moreover, the number of phrase sets existing in the same page is registered therewith as a relation value by referring to the occurrence pattern data (1013). Thus, creation of the knowledge database to be created is completed (1014).

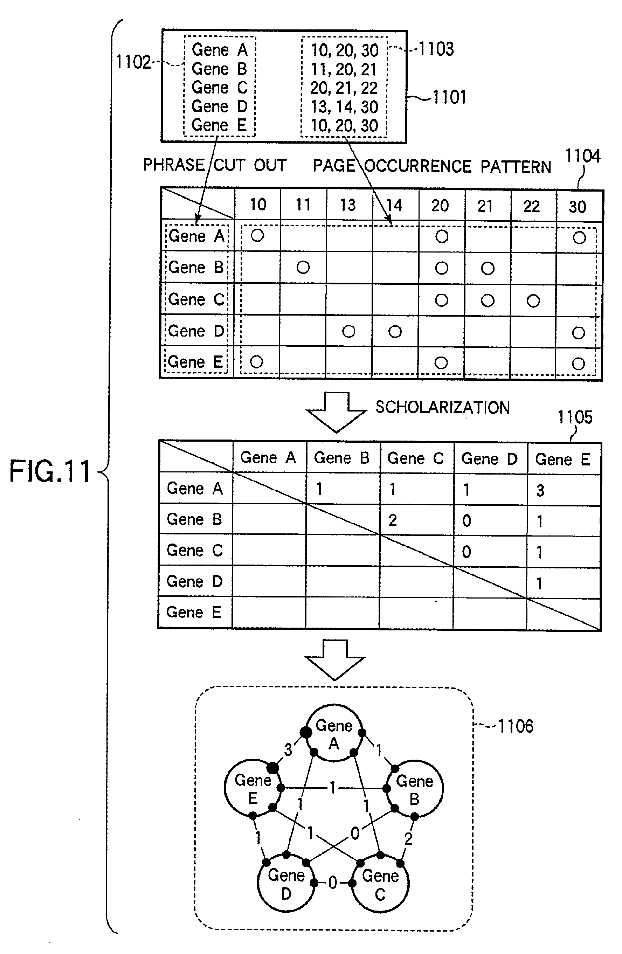

Fig. 11 illustrates a method, in which an index is set as a knowledge resource, phrase objects are cut out therefrom, and relations are set therebetween. First, from the index 1101 as a knowledge resource, the phrase portion 1102 and the page number portion 1103 are extracted, respectively, the phrases are cut out, and the occurrence pattern data (1104) composed of a page occurrence pattern is generated (processing of 1001 and 1011 in Fig. 10). Based on the occurrence pattern data, phrases are set as the phrase objects, and a relation value is obtained by counting the number of occurrence of two phrases in the same page (1105) (processing of 1012 and 1013 in Fig. 10). The processing in (1105) is illustrated as in 1106.

Moreover, as another method for extracting phrase objects from a knowledge resource as lingualized knowledge, for example, there is a method, in which previously designated optional phrases (e.g., terms, idioms and clauses) are used as master data of the phrase objects, and the same phrases are extracted from a target knowledge resource. In this case, first, an index indicating the existence of the phrases in a knowledge unit can be created by the designated phrase, then cutting out of the phrase objects and setting of the relations can be carried out by the same method as the above-described case where the index of a book is set as a knowledge resource.

Moreover, as a method for obtaining a relation value from an occurrence pattern in a knowledge unit, there exists another useful method, in which, not only the existence of the phrase objects in each knowledge unit shown in the above two examples, but existing amounts are accumulated and subjected to cluster analysis as existing profiles in the respective knowledge units, whereby a similarity between the existing profiles of the phrase objects in the respective knowledge units are quantified, and the quantified similarity is set as a relation value between the objects. By use of this method, in the method for obtaining a relation value from an occurrence pattern in a knowledge unit, particularly, in the case of targeting a knowledge unit having a relatively large amount of information such as an academic treatise and a Web page, dilution of the information can be avoided.

In the knowledge database constituted of the phrase objects, an information density of the knowledge greatly depends on selection of the lingualized knowledge as a knowledge resource. Specifically, redundant information and sundry information, which are included in the lingualized knowledge, becomes noise information in the knowledge database and hinders extraction of semantic information from the knowledge database. The knowledge resource for use in constructing the knowledge database is selected in accordance with a purpose, thus making it possible to constitute a knowledge database having a small amount of noise information.

Specifically, in the case of targeting an index of a book such as a textbook, a handbook and a manual as a knowledge resource of the lingualized knowledge, it is made possible to fetch only phrases carefully selected by an author or an editor of the book into the knowledge database. A frequently appearing word such as a "DNA" and a "gene" that will be a noise in a general lingualized knowledge is not employed as a single phrase in the index but appears only as a phrase constituting the knowledge. Therefore, such a frequently appearing word can be utilized as a phrase object. Moreover, in the case of targeting a table of contents of a book such as a textbook, a handbook and a manual as a knowledge resource of the lingualized knowledge, there is described only a fact based on knowledge at the time when the book is written. Therefore, a strong relation can be obtained selectively together with the carefully selected phrases.

Moreover, in the case of targeting a title of an academic treatise as a knowledge resource of the lingualized knowledge, as a feature of an academic treatise in medicine/biology, a title thereof includes the most important element of the knowledge proved and explained by the treatise; therefore, a strong relation can be obtained selectively together with the carefully selected phrases. It is possible to acquire the title of the academic treatise through a computer network. Moreover, it is possible to maintain a knowledge database reflecting up-to-date knowledge by a system, in which an up-to-date academic treatise is periodically acquired, phrase objects and relations are extracted from a title of the treatise by automatic processing, and the knowledge database is updated.

Moreover, a body text of a textbook, an academic treatise or the like is targeted as a knowledge resource of the lingualized knowledge, thus making it possible to obtain a uniform phrase object. Specifically, such a resource includes synonyms little, which hinder information processing in the general lingualized knowledge. Accordingly, dilution of the knowledge information caused by the synonyms can be avoided. For example, while terms such as a "microarray", a "DNA chip" and a "biochip" imply the same phenomenon in some cases, the terms are generally unified in such a resource. It is possible to acquire the academic treatise through a computer network. Moreover, it is possible to maintain a knowledge database reflecting up-to-date knowledge by a system, in which an up-to-date academic treatise is periodically acquired, phrase objects and relations are extracted from a body text by automatic processing, and the knowledge database is updated.

DNA sequence objects and protein sequence objects are extracted from a knowledge resource including lingualized knowledge together with a DNA sequence and a protein sequence. Typically, the DNA sequence and the protein sequence are converted into data together with identifiers for identifying the DNA sequence and the protein sequence and lingualized knowledge describing an origin and a function of the DNA sequence and the protein sequence. From such a knowledge resource, the DNA sequence or the protein sequence is extracted as the DNA sequence objects or the protein sequence objects, and relations are set therebetween. Moreover, identifiers concomitant with the sequences are extracted as phrase objects, and a value for "regarding two target objects as the same" is set as a relation between the corresponding DNA sequence objects or protein sequence objects. Moreover, relations are set between the other phrase object extracted from the lingualized knowledge and a phrase object having an identifier of the DNA sequence as an object value or a phrase object having an identifier of the protein sequence as an object value. Furthermore, relations are set between the other relevant phrase objects extracted from the lingualized knowledge. Thus, a knowledge database including both of the phrase objects and the DNA sequence objects or the protein sequence objects is constructed. Since this knowledge database includes the phrase objects, it is possible to readily merge the knowledge database into a knowledge database including the other phrase objects.

In the case of setting a database accumulating DNA sequence information or protein sequence information as a knowledge resource, the above-described operation is carried out for DNA sequence entry units or protein sequence entry units, and knowledge databases generated in the entry units are entirely merged to obtain a master knowledge database. It is possible to acquire, through a network, the knowledge resource including both of the lingualized knowledge and the DNA sequence or the protein sequence, alternatively both of the sequences. Moreover, it is possible to maintain a knowledge database reflecting up-to-date knowledge in a manner that up-to-date information is periodically acquired, the DNA sequence objects or the protein sequence objects, the phrase objects and the relations are extracted therefrom, and a knowledge database for the periodically acquired knowledge resource is created to be merged into the master knowledge database.

In a knowledge database constituted of image objects, for example, results of characteristic value analysis between the image objects and image acquiring statuses are set as relation values. For example, in images acquired in time series, the closer the times of acquiring the images are, the higher the relation value therebetween is set. A knowledge database having relation values between image objects prepared according to a purpose is merged into a knowledge database including the other image objects, thus making it possible to provide characteristic relation values between the image objects. For example, a knowledge database having a series of images capturing a change in time series in growth, aging or lesion of an organic tissue is prepared and merged into a knowledge database including organic tissue images acquired from a plurality of samples, thus making it possible to construct a knowledge database including a quantitative relation seen between the organic tissue images of the respective samples.

The image objects are extracted from a knowledge resource including the images and lingualized knowledge related thereto. For example, from an image file having an identifier for identifying an image, the image is extracted as an image object, and the identifiers are extracted as phrase objects. Moreover, phrase objects are extracted from lingualized knowledge coupled to the image by the identifiers and describing the contents of the image. Then, a value for "regarding two target objects as the same" is set as a relation between the image object and the phrase object having the identifier of the image as an object value. Moreover, between the phrase objects extracted from the lingualized knowledge coupled to the image by the identifiers and describing the contents of the image and between the relevant phrase object and the phrase object having the identifier as the object value, relations are set. Since a knowledge database including these objects and relations includes the phrase objects, the knowledge database can be readily merged into a knowledge database including the other phrase objects.

Such a knowledge database including both of the phrase objects and the image object is generated also by setting a Web page as a knowledge resource. A URL for identifying the Web page, a URL for identifying an image included in the Web page, lingualized knowledge included in the Web page and the like are set as phrase objects, and relations are set between these phrase objects and the image (the image object) in the Web page, thus making it possible to readily merge the relevant knowledge database into a knowledge database including the other phrase objects.

As a knowledge resource, knowledge expressed in a structured description language such as an HTML and an XML is also usable. In this case, in accordance with an expression format of the knowledge resource and definition thereof, information to be extracted as objects, types of the objects and relation to be set therebetween are defined and utilized according to the expression format and the definition.

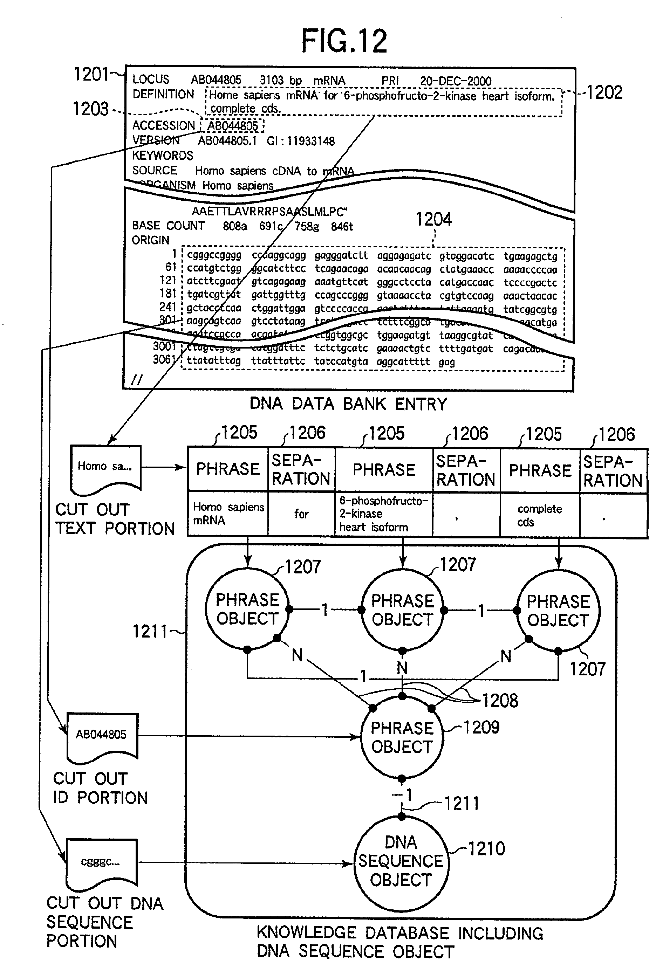

Fig. 12 is a view showing, as an example of generating a knowledge database having different types of objects, an example of a method for cutting out DNA sequence objects and phrase objects related thereto from a DNA database entry as a knowledge resource and for setting relations therebetween.

The typical DNA database entry 1201 includes the portion 1202 for defining the DNA, the identifier 1203 for identifying the DNA and the DNA sequence 1204. After the respective portions are cut out from the DNA database entry, the portion 1202 for defining the DNA is decomposed into the phrases 1205 by the phrase-separating letter strings 1206 similarly to the title of the treatise, and the phrase objects 1207 and the relations therebetween are generated. Here, the relation values are set as 1. The identifier 1203 for identifying the DNA is set as a phrase object as it is, and relations are set between the identifier 1203 as a phrase object and the entire phrase objects generated from the portion for defining the DNA in the same DNA database entry. Here, the relation values are set as N. Finally, the DNA sequence 1204 is set as the DNA sequence object 1210, and the relation 1211 is set between the DNA sequence object 1210 and the phrase object generated from the identifier 1203 for identifying the DNA, the relation 1211 being for regarding the two objects as the same. Here, -1 is set as a value for regarding the two objects as the same.

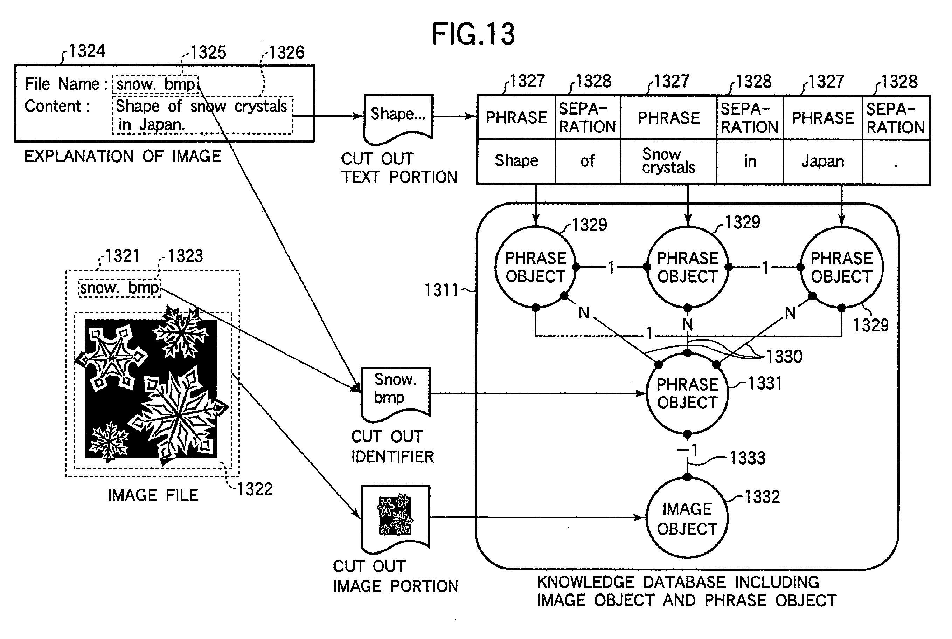

Fig. 13 is a view showing, as an example of generating a knowledge database having different types of objects, an example of a method for cutting out an image object and phrase objects related thereto from an image file and lingualized knowledge for describing the image as a knowledge resource and for setting relations therebetween.

The image file 1321 includes the image 1322 and the file name 1323 as an identifier of the image. Moreover, in the description 1324 of the image, there exist the file name 1325 of the image file as an identifier of the image and the lingualized knowledge 1326 for describing the image. After the respective portions are cut out, the lingualized knowledge for describing the image is decomposed into the phrases 1327 by the phrase-separating letter strings 1328 similarly to the title of the treatise, and thus the phrase objects 1329 and the relations therebetween are generated. Here, the relation values are set as 1. As an identifier for identifying the image, the file name is set as the phrase object 1331, and the relations 1330 are set between the phrase object 1331 and the entire phrase objects generated from the lingualized knowledge for describing the image. Here, the relation values are set as N. The image is set as the image object 1332, and the relation 1333 is set between the image object 1332 and the phrase object 1331 generated from the identifier for identifying the image, the relation being for regarding the two objects as the same. Here, -1 is set as a value for regarding the two objects as the same.

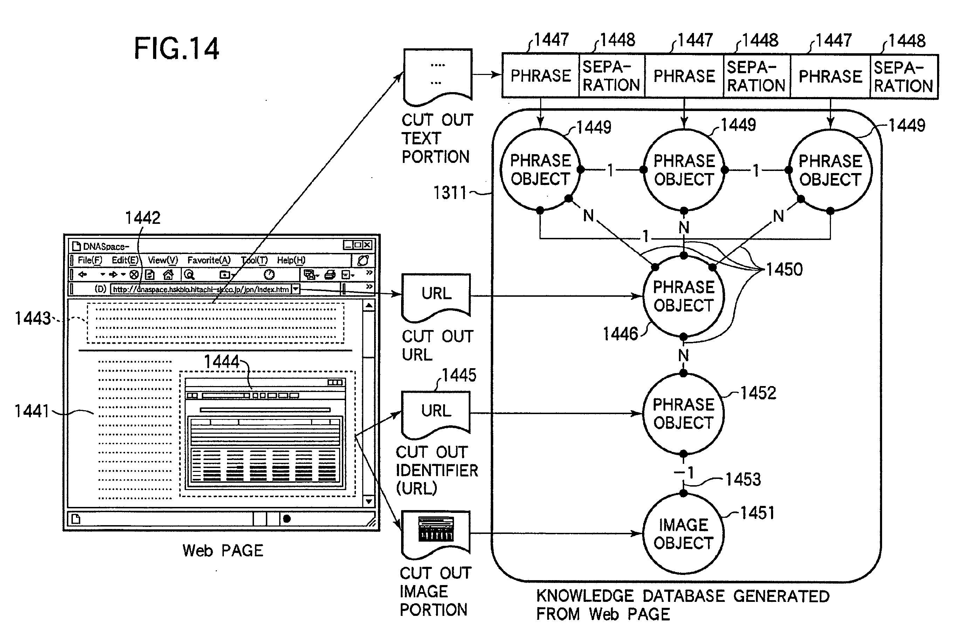

Fig. 14 is a view of an example of generating a knowledge database from a Web page. The Web page 1441 includes the URL 1442 of the Web page, the lingualized knowledge 1443 included in the Web page, the image 1444, the URL 1445 thereof and the like. After the respective portions are cut out, the URL of the Web page is set as the phrase object 1446. And the lingualized knowledge included in the Web page is decomposed into the phrases 1447 by the phrase-separating letter strings 1448 similarly to the title of the treatise, and thus the phrase objects 1449 and the relations therebetween are generated. Here, the relation values are set as 1. The image is set as the image object 1451. And, as an identifier for identifying the image, the URL of the image is set as the phrase object 1452, and the relation 1453 is set between the image object 1451 and the phrase object 1452, the relation being for regarding the two objects as the same. Here, -1 is set as a value for regarding the two objects as the same. The relations 1450 are set between the phrase object generated from the URL of the Web page and the phrase object generated from the URL of the image included in the Web page and between the phrase object generated from the URL of the Web page and the entire phrase objects generated from the lingualized knowledge included in the Web page. Here, the relation values are set as N.

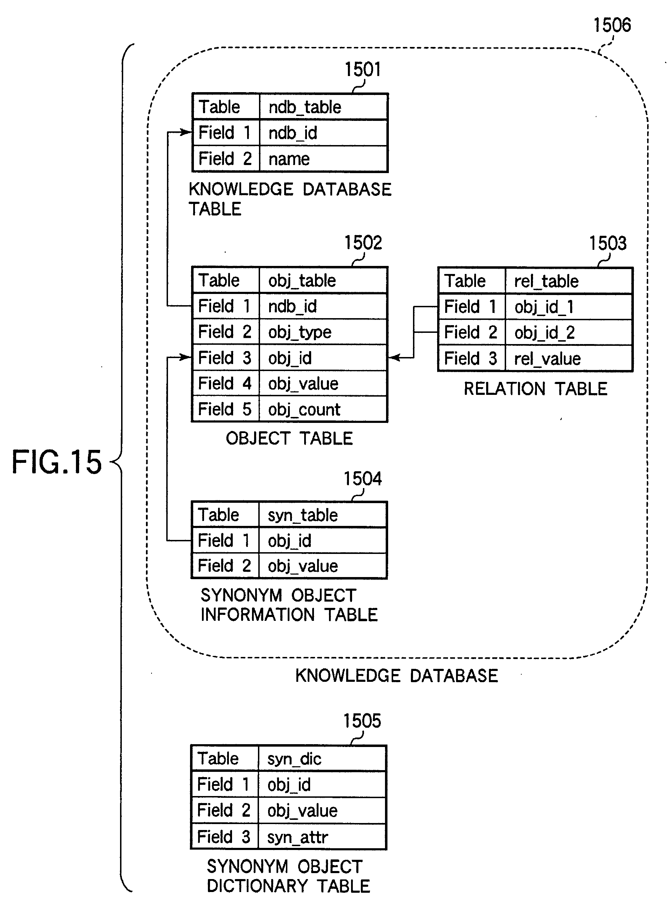

Fig. 15 is a view showing information concerning synonym objects and a system for storing a synonym object dictionary as a data storage system in a knowledge database and information concerning the knowledge database with a relational database taken as an example.

In this data storage system, a plurality of knowledge databases can be stored. Specifically, one knowledge database 1506 is identified from the other by one record of the knowledge database 1501. To each knowledge database, the knowledge database identifier (ndb_id) as a unique ID is imparted. It is made possible to identify as to which knowledge database each record of the object tables belongs to by imparting the knowledge database identifier thereto. In such an object record, besides the knowledge database identifier, it is possible to store the object type (obj_type), the object identifier (obj_id), the object value (obj_value) and the object count (obj_count). The object identifier is set as a unique value in the knowledge database. And even in different knowledge databases, objects having the same object identifier are defined to have the equal value. In the object type, stored is information indicating a type of the object such as a phrase object and a DNA sequence object. In the object value, an object value is stored as a text or binary code. In the object count, occurrence frequencies of a target object in a knowledge resource are recorded in order to be used for standardization of relation values in the phrase objects.

It is made possible to store two object identifiers in the relation table 1503 and to identify as to which objects are defined by a relation stored therein. Moreover, it is made possible to store the relation value (rel_value). In the synonym object information table, stored are the object identifier indicating an object having a synonym and the synonym value. Since the value of the synonym is equivalent to that of the object, a field name being the same as the value of the object is given thereto.

Moreover, the synonym object dictionary table 1505 is prepared. In the synonym object dictionary table 1505, stored are the object identifier for identifying a group of synonyms, the value of the synonym and additional information of the synonym (syn_attr). Since the identifier of the synonym and the value of the synonym are information equivalent to the object, field names being the same as the object identifier and the value of the object are given thereto. In the synonym additional information, for example, stored is information as to whether or not a synonym in the target record is a representative synonym in the group of synonyms.

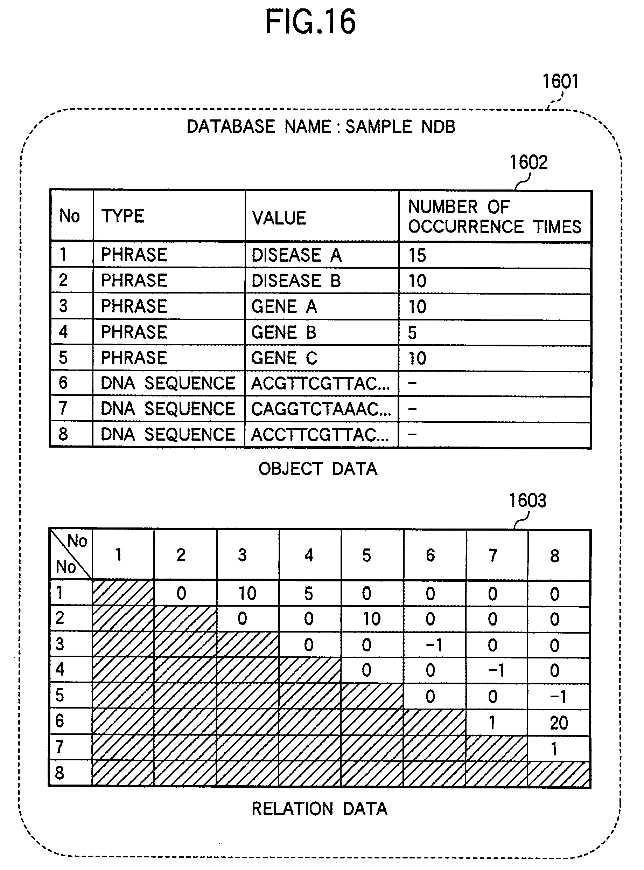

Fig. 16 is a view showing an example of information to be stored in a knowledge database. As shown in Fig. 16, as the information 1602 concerning the objects, the numbers, the types, the values and the occurrence frequencies of the objects are given. As the relation 1603, a matrix of the relation values is given by the numbers of the objects. As the name of the knowledge database 1601 including all the above, "Sample NDB" is given.

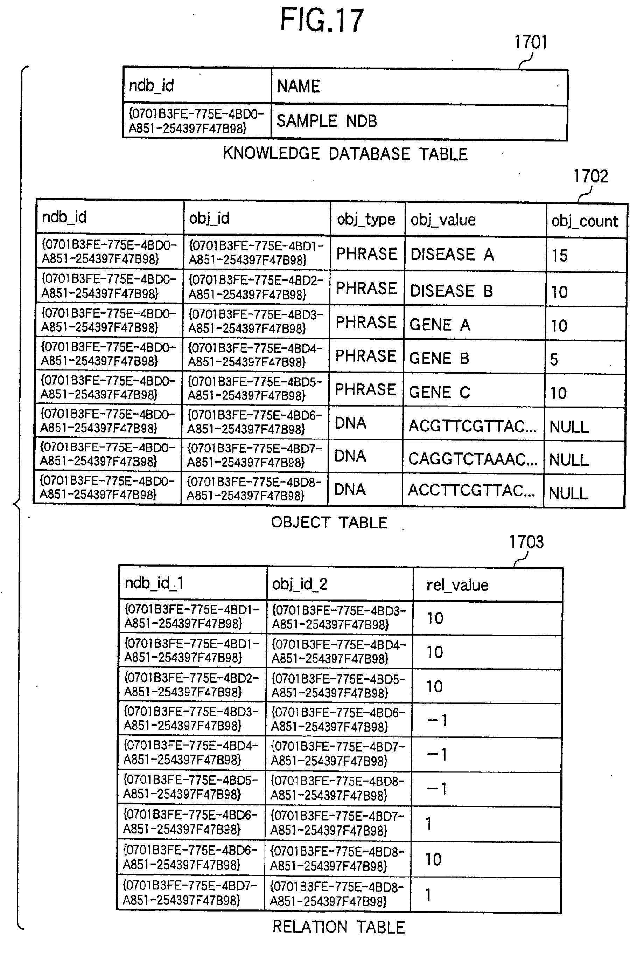

Fig. 17 is a view showing an example of storing the information shown in Fig. 16 by the system shown in Fig. 15. In the knowledge database table 1701, stored is one record indicating the target knowledge database having the knowledge database identifier and the knowledge database name. In the object table 1702, stored are the knowledge database identifiers indicating the knowledge databases to which the objects belong, the object identifiers, the object types, the object values and the object counts. In each row of the relation table 1703, stored are two object identifiers for identifying a relation therebetween and the relation value. Here, a regulation is made so as not to store a relation having a value of zero in the relation table.

A plurality of phrase objects having the equal value are chosen from a plurality of knowledge databases, and relation values between such phrase objects are extracted and compared, thus a difference in knowledge constitution between the plurality of knowledge databases can be known. Here, with regard to the target phrase objects, optional interesting phrase objects are selected by the user, alternatively, phrase objects regarding relations having relation values equal or more than an optional value are selected, which are included in the plurality of target knowledge databases. Alternatively, phrase objects are selected by combining the above two systems. As a method of comparison, for example, a two-dimensional graph is written, where the target relation designated by the set of the two phrase objects is taken on one axis, the relation value is taken on the other axis, and broken lines different in color, line type and marker for each knowledge database are drawn. In displaying the graph, the axis of the target relation is sorted by the relation values of the target relations in a certain knowledge database, thus making it possible to facilitate the comparison with the values of the other knowledge databases.

Moreover, in the knowledge database comparison for a knowledge database significant in that information is arrayed in time series, for example, for the knowledge database generated from information as a knowledge resource collected for every certain period of time, for example, for every year and for every month, a two-dimensional graph is written, where the knowledge database sorted in time series is taken on one axis, the relation value is taken on the other axis, and broken lines different in color, line type and marker for each target relation designated by the set of the two phrase objects are drawn. By such drawing of the graph, it is made possible to readily observe a change of the knowledge in the knowledge resource in time series.

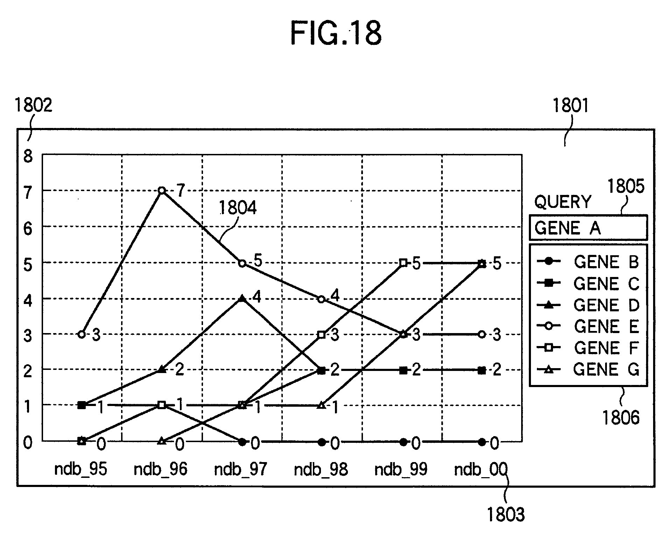

Fig. 18 is a view showing an example of a graph indicating changes of relation values related to a certain object among a plurality of knowledge databases.

In this drawing, in six knowledge databases (ndb_95, ndb_96, ndb_97, ndb_98, ndb_99 and ndb_00), objects having strong relations with the object 1805 having a value of "gene A" in any of the knowledge databases (a target object) are extracted and displayed on the graph. The relation value 1802 is taken for the axis of ordinates, and the knowledge database 1803 is taken for the axis of abscissas. With regard to the six objects 1806 having relation values larger than zero with the object having the value of the "gene A" in any of the knowledge databases, the values are represented by the line graph 1804, respectively. By this method, it can be known how the relations of the other objects with respect to the focused object therewith differ in the different knowledge databases. Moreover, when the target knowledge database is created from a knowledge resource changed in time series, the knowledge databases on the axis of abscissas are arrayed in time series in accordance with the original knowledge resources, and thus the change of the relations in time series with regard to the objects related to the focused object can be seen.

In the event of performing an operation of the knowledge databases including the phrase objects, which are generated from the different knowledge resources, alternatively, in the event of using the knowledge databases focusing on a difference in information between the knowledge databases, for example, comparison of the knowledge databases, in order to even, between the knowledge databases to be operated or used, effects and information amounts of the relations between the phrase objects, the relation values are standardized between the knowledge databases to be operated or used prior to the operation or the use. Specifically, absolute values of the relation values, which originate from sizes of the knowledge resources used for generating the respective knowledge databases and biases of the information included in the knowledge resources are converted into relative values to the sum of the relation values in the respective knowledge databases or into relative values to the focused relation, thus enabling the operation or the use, which correctly reflects the information of the target knowledge database.

As a method for standardizing relation values, there is a method for standardizing relation values based on the occurrence frequencies of the phrase objects extracted from the knowledge resources used for generating the knowledge databases. For example, in the generation of the knowledge databases from the knowledge resources of the lingualized knowledge, the number of extraction for each phrase object is recorded. Thus, in the event of the operation or the use of the knowledge databases, in the target knowledge databases, the sums of the numbers of extracting the entire phrase objects are obtained; alternatively, the sums of the numbers of extracting the focused phrase object are obtained. Then, the entire relation values are standardized so that the values of the sums can be equal between the target knowledge databases. In the case of using this method for the sums of the knowledge databases, the sums of the numbers of extracting the phrase objects standardized in both the knowledge databases as operation sources are recorded in the knowledge database as an operation result, thus making it possible to subsequently standardized the sum with the other knowledge database.

Moreover, there is another standardization method, which focuses on the relation values between the phrase objects included in the knowledge databases to be operated or used. For example, in the event of the operation or the use of the knowledge databases, in the target knowledge databases, the sum total of the relation values between the phrase objects included in the respective knowledge databases are obtained, alternatively, the sum total of the relation values between the focused phrase object and the phrase objects related thereto are obtained, and alternatively, one focused relation value is obtained. Then, the entire relation values are standardized so that the values of the above-described sums can be equal between the target knowledge databases.

Standardization is performed based on the occurrence frequencies of the focused phrase objects in the knowledge resources or based on the values of the focused relations in the knowledge databases. Thus, enabled is more accurate processing for the biases of the knowledge between the knowledge databases in the entire knowledge databases or on a periphery of the focused phrase objects or relations.

In displaying the information concerning the objects included in the knowledge databases, selection of the target objects is carried out by designating or searching the objects included in the knowledge database. In designating the objects, the object values are inputted. For example, in inputting values for designating the phrase objects, in accordance with input of object values desired to be designated, the phrase objects having head-matching and equal values are displayed as candidates thereof on a list, and selection of the phrase objects is carried out therefrom. A user interface capable of selecting the objects in the above-described manner is desired. In searching the objects, it is recommended that the search be performed based on the object values by a searching method prepared for each type of the object. For example, with regard to the phrase objects, a search for the phrases by use of the normalized expression may be prepared. With regard to the DNA sequence objects and the protein sequence objects, a search by use of the homology search may be prepared. Moreover, with regard to the image objects, a search by use of the pattern matching of the images may be prepared. With regard to one or a plurality of objects selected by the designation or the search, a method for displaying information related thereto on a list or graphically may be prepared. As the information displayed on a list, enumerated are types of the objects, values of the objects, the sum of the relation values of the relations related to the objects, the numbers of the objects having relations of the designated value or higher with the relevant object, and the numbers of objects having values for regarding the objects as the same. Moreover, in the case of the phrase objects, as the information, the number of extracting the phrase objects from the knowledge resources is enumerated. When the designated object is one, with regard to the objects having the relations of the designated value or higher with the relevant objects, similar information may be displayed simultaneously on a list. In the graphical display, for example, markers indicating the target objects are arrayed on a circumference at an equal interval, and lines indicating the relations are drawn between the markers. Sizes of the relation values are shown by line width, thus making it possible to readily identify the relations between the entire target objects.

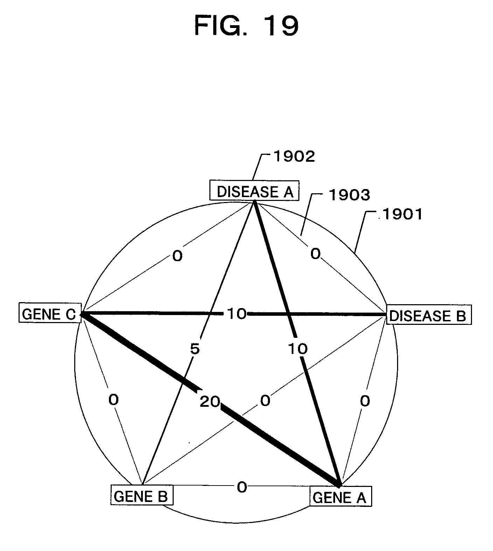

Fig. 19 is a view showing an example of a graphical display for displaying the relations of the plurality of objects designated in the knowledge databases. Optional objects included in the knowledge databases are selected by the designation or the search, and such objects 1902 selected are disposed on the circumference 1901 at an equal interval. Then, the entire objects are connected by the lines 1903 representing the relations. The sizes of the relation values are shown by the line width, and the relation values are displayed on the lines according to needs, and thus degrees of strength in relation between the objects are designated. Moreover, in the case of simultaneously displaying a large number of objects, only the relations having relation values of a designated value or higher are displayed, thus simplifying the display.

These knowledge databases are basically used for displaying information related to keywords to be investigated, by giving the keywords to knowledge databases constructed according to a purpose. In this case, as a method for extracting the information from the knowledge databases constructed according to the purpose, a method for hierarchically selecting objects (target objects) having strong relations with one or a plurality of objects (query objects) corresponding to the keywords designated is effective.

Specifically, relations related to the query objects are sorted in order from the one having a higher relation value, and the preset number of relations are selected sequentially from the top, thus the objects (target objects) related by the selected relations are obtained in a first hierarchy. Next, similar processing is executed with the target objects obtained in the first hierarchy taken as new query objects, and thus the next target objects are obtained in a second hierarchy. The target objects are hierarchically obtained by the number designated, and thus objects hierarchically related to the query objects given in the first hierarchy can be obtained. In this method, in the event of selecting the relations related to the query objects, the minimum value of the relation values to be selected is set, and deduction is carried out for the relations based on the minimum value, thus a network of the hierarchical objects, which is constituted of only principal relations, can be obtained.

Alternatively, the knowledge databases are basically used for displaying information related to a group consisted of a plurality of keywords. In this event, a method for extracting information from the knowledge databases constructed according the purpose is carried out as below. Specifically, when the plurality of objects corresponding to the designated keywords are set as first query objects, prior to acquiring the target objects in the first hierarchy, the entire query objects and the relations related thereto are merged and set as one object, and this one object is set as a new query object, then the objects are hierarchically selected. According to this method, it is possible to obtain objects connected by relations having averagely high relation values for the objects belonging to the group. For example, in the case where keywords to be investigated are many, it is useful to obtain objects in which keywords have a strong relation as an aggregate.

As methods for displaying the objects hierarchically having the relations, which are obtained by the above-described method, list display on a table format and graphical display are prepared. In the graphical display, when the query object given as the first hierarchy is one, the target objects are arrayed on concentric circles having the query object as a center and radii becoming larger for later hierarchies, and the query object and the target objects are connected by lines, thus making it possible to effectively display the relations and the hierarchies between the objects. In this case, the sizes of the relation values may be expressed by the line width. Moreover, the same objects are not displayed duplicately on the display. Specifically, the objects and the relations are displayed from a low hierarchy to a high hierarchy, an object already displayed on a lower hierarchy or the same hierarchy is not newly displayed, and the relations are connected between the objects already displayed.

Moreover, whichever the query object given as the first hierarchy may be one or plural, the objects are arrayed in a horizontal or vertical direction for each hierarchy, and the query object and the target objects are connected by lines, whereby the relations and the hierarchies between the objects can be effectively displayed. In this case, the sized of the relation values are expressed by the line width. Moreover, the same objects are not displayed duplicately on the display. Specifically, the objects and the relations are displayed from a low hierarchy to a high hierarchy, an object already displayed on a lower hierarchy or the same hierarchy is not newly displayed, and the relations are connected between the objects already displayed.

In the graphical display of the objects having the relations hierarchically, it should be made possible to set one or a plurality of optional objects in a selection state from the displayed objects. Specifically, it is desirable that improvement of the display be enabled by dragging and re-disposing the optional objects manually. Moreover, a user interface is prepared, which is for changing setting of the number of target objects to be displayed on the next hierarchy and the minimum relation value with regard to the selected objects and for reflecting the changed setting on the display. Furthermore, it should be made possible to set the selected objects as query objects and thus to extract and display new knowledge information from the designated knowledge databases. Moreover, in the case where the plurality of objects are selected, it should be made possible to register the objects as a synonym object group and thus to reconstitute the knowledge databases. In this event, it is made possible to designate a representative object among the selected objects.

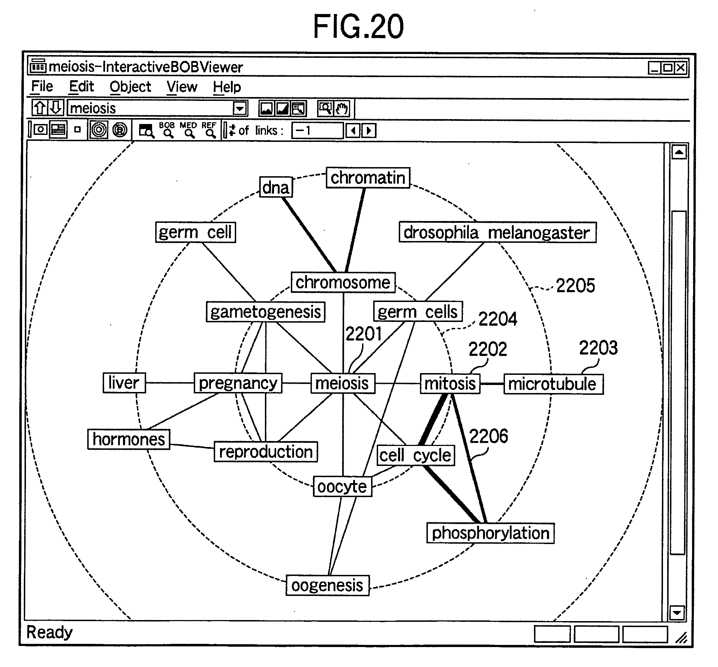

Fig. 20 is a view showing an example of a graphics display interface for, in a knowledge database, displaying one designated object (query object), objects hierarchically related thereto and mutual relations therebetween on a form expanding concentrically.

The query object 2001 is displayed on the center. The objects 2002 having relations of a designated value or higher with the query object 2001 are extracted by the designated number from the knowledge database and are set as the objects of the first hierarchy. The objects 2002 are disposed at an equal interval on the circumference 2004 with the query object taken as a center. Subsequently, the objects 2003 having relations of a designated value or higher with the respective objects of the first hierarchy are extracted by the designated number from the knowledge database. Then, the objects 2003 are set as objects of the second hierarchy, and are disposed at an equal interval on the circumference 2005 larger in radius than the circumference 2004 of the first hierarchy with the query object taken as a center. In this event, the objects already displayed on the first hierarchy are not included in the second hierarchy. Moreover, each object of the second hierarchy is made to be disposed as close to the object of the first hierarchy as possible, with which each object has a close relation. In such a manner, the objects are extracted and displayed hierarchically for the designated hierarchies.

Moreover, the entire displayed objects are connected by the lines 2006 representing the relations therebetween. The sizes of the relation values are shown by the line width, and the relation values are displayed on the lines according to needs, thus degrees of strength in relation between the objects are designated. In the case of simultaneously displaying a large number of objects, only lines showing the relations having relation values of a designated value or higher may be displayed, thus simplifying the display. Desirably, it should be made possible to select the displayed objects and to move the selected objects to easily viewable positions by dragging. When the objects are moved, the lines showing the relations are simultaneously moved. With regard to the selected objects, it should be desirably possible to increase or decrease the number of objects to be displayed on the next hierarchy. Alternatively, it should be made possible to delete the selected objects themselves from the display. Moreover, it should be made possible to set the selected objects as query objects and thus to extract and display new knowledge information from the designated knowledge databases.

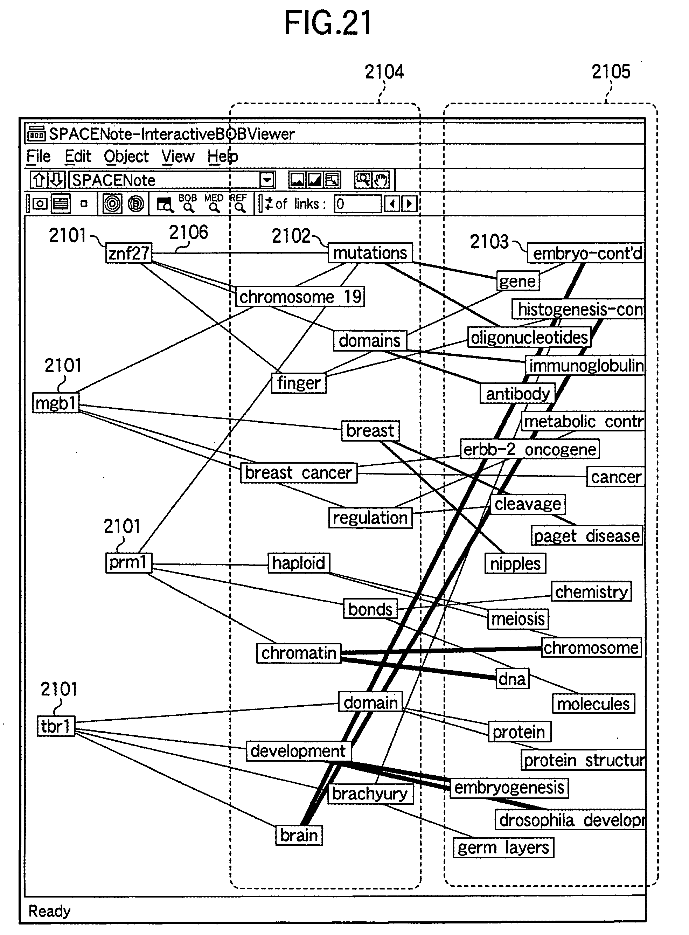

Fig. 21 is a view showing an example of a graphics display for displaying designated one or plurality of objects, objects hierarchically related thereto, and mutual relations therebetween on a form of expanding parallel.

The query objects 2101 are displayed linearly at an equal interval. In displaying the objects, in order to improve easiness to see the display when a large number of objects are displayed, the objects may be displayed alternately. The query objects 2102 having relations of a designated value or higher with each query object 2101 are extracted by the designated number from the knowledge databases and are set as the objects of the first hierarchy. The objects 2102 are disposed at an equal interval on the position 2104 parallel to the query objects. Subsequently, the objects 2103 having relations of a designated value or higher with the respective objects of the first hierarchy are extracted by the designated number from the knowledge databases. Then, the objects 2103 are set as objects of the second hierarchy, and are disposed on the position 2105 more separate than the first hierarchy parallel to the query objects from the query objects. In this event, the objects already displayed on the first hierarchy are not included in the second hierarchy. Moreover, each object of the second hierarchy is made to be disposed as close to the object of the first hierarchy as possible, with which each object has a close relation.

In such a manner, the objects are extracted and displayed hierarchically for the designated hierarchies. Moreover, the entire displayed objects are connected by the lines 2106 representing the relations therebetween. The sizes of the relation values are shown by the line width, and the relation values are displayed on the lines according to needs, thus degrees of strength in relation between the objects are designated. In the case of simultaneously displaying a large number of objects, only lines showing the relations having relation values of a designated value or higher may be displayed, thus simplifying the display. Desirably, it should be made possible to select the displayed objects and to move the selected objects to easily viewable positions by dragging. When the objects are moved, the lines showing the relations are simultaneously moved. With regard to the selected objects, it should be desirably possible to increase or decrease the number of objects to be displayed on the next hierarchy. Alternatively, it should be made possible to delete the selected objects themselves from the display. Moreover, it should be made possible to set the selected objects as query objects and thus to extract and display new knowledge information from the designated knowledge databases.

This method for using knowledge databases can be applied to a method, in which information obtained resultantly from a search of databases related to medicine/biology is to be investigated, objects corresponding to the information are set as query objects, and the information is extracted from knowledge databases. In this event, the information obtained resultantly from the search of the databases related to medicine/biology includes phrases describing technical terms, molecule names, gene names, biological genera, URLs and the like, and includes images such as molecule structures including DNA sequences and protein sequences, organic tissue images and the like. For example, among information obtained resultantly from a homology search of the DNA sequences or the protein sequences in the DNA sequence database or the protein sequence database, accession numbers, DNA sequence names, protein names and the like are given to the knowledge databases including the phrase objects. Then, the phrase objects having the same object value are set as query objects, and the information thereby obtained is displayed. Alternatively, among the information obtained resultantly from the homology search, the DNA sequences or the protein sequences are given to the knowledge databases including the DNA sequence objects or the protein sequence objects. Then, the DNA sequence objects or the protein sequence objects, which have high object values in homology are set as query objects, and the information thereby obtained is displayed.

Moreover, there is a method, in which information obtained resultantly from medical/biological experiments or data analyses is to be investigated, objects corresponding to the information are set as query objects, and the information is extracted from knowledge databases. In this event, the information obtained resultantly from the search of the databases related to medicine/biology includes phrases describing technical terms, molecule names, gene names, biological genera, URLs and the like, and includes images such as molecule structures including DNA sequences and protein sequences, organic tissue images and the like. For example, in DNA sequence deciding experiments, DNA sequences decided by a DNA sequencer are given to the knowledge databases including the DNA sequence objects or the protein sequence objects. Then, the DNA sequence objects or the protein sequence objects, which have high object values in homology, are set as query objects, and the information thereby obtained is displayed. Moreover, gene name clusters obtained resultantly from cluster analyses of gene occurrence data are given to the knowledge databases including the phrase objects. Then, phrase objects having the same value as each gene name are set as query objects, and information thereby obtained is displayed.

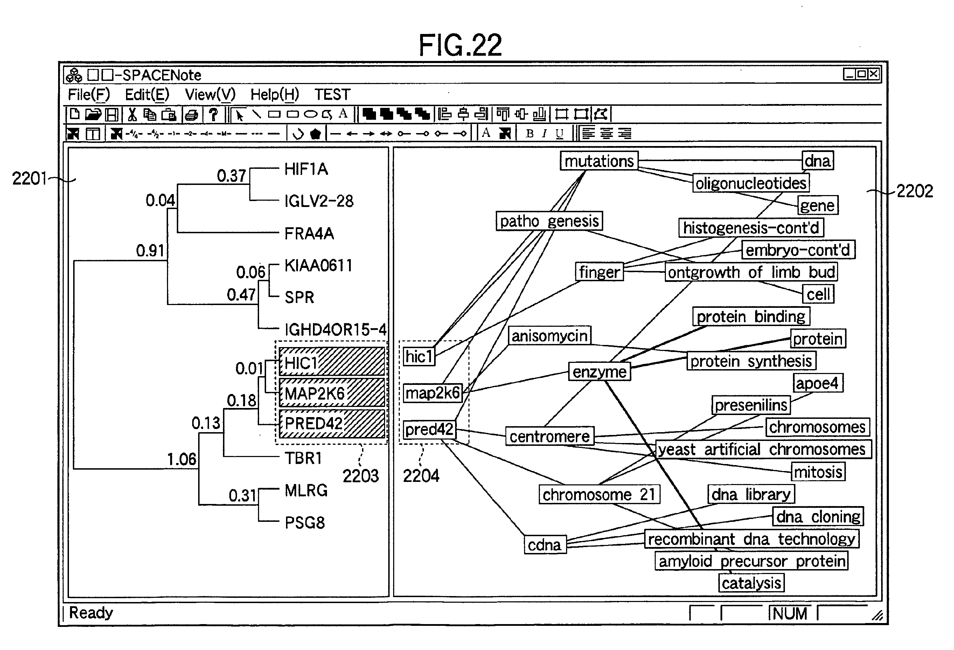

Fig. 22 is a view showing an application example of knowledge databases, in which clusters of phrase objects having the same object values as gene names are set as query objects, the clusters being obtained resultantly from analyses for gene occurrence profiles, and information concerning objects hierarchically related to the clusters is displayed by the display method shown in Fig. 21.

In the analyses for the gene occurrence profiles, the gene clusters classified by the cluster analyzing application 2201 are selected (2203) and delivered to the knowledge database application 2202. Then, the phrase objects having the same object value are set as the query objects 2204, and the objects and the relations are hierarchically extracted from designated knowledge databases, which are then displayed by a method similar to that in Fig. 21. In such a manner, by application of the knowledge databases, knowledge existing in the existing knowledge resources can be obtained from the results of the medical/biological experiments and the data analyses.

As described above, according to the present invention, knowledge of medicine/biology can be accumulated and utilized by structuralization, thus enabling explanation of the knowledge, expression of the knowledge, finding of the knowledge and utilization of the knowledge by use of an information system.

## Claims

1. A knowledge database comprising:
a plurality of phrase objects having phrases included in natural language information as object values; and
relations between the phrase objects, the relations having values expressing the relations between the phrase objects quantitatively as relation values.

2. A knowledge database comprising:
a plurality of objects having any of DNA sequences and protein sequences as object values; and
relations between the objects, the relations having values obtained by any of homology calculation and experimental measurement for any of relations between the DNA sequences and relations between the protein sequences to be expressed quantitatively as relation values.

3. A knowledge database comprising:
a plurality of objects; and
relations between the objects,
wherein the objects include any of DNA sequence objects having DNA sequences as object values and protein sequence objects having protein sequences as object values, and phrase objects having phrases included in natural language information related to any of the DNA sequences and the protein sequences as object values, and
any of relations between the DNA sequence objects and the phrase objects and relations between the protein sequence objects and the phrase objects have relation values previously defined.

4. A knowledge database comprising:
a plurality of objects having images as object values; and
relations between the objects, the relations having values expressing the relations between the objects quantitatively as relation values.

5. A knowledge database comprising:
a plurality of objects; and
relations between the objects,
wherein the objects include image objects having images as object values and phrase objects having phrases included in natural language information related to the images as object values, and
relations between the image objects and the phrase objects have relation values previously defined.

6. A knowledge database comprising:
a plurality of objects; and
relations between the objects,
wherein the objects include phrase objects having phrases including terms for use in a medical field and/or a biological field as object values, and
the relations have values expressing the relations between the phrase objects quantitatively as relation values.

7. The knowledge database according to claim 6,
wherein a synonym object dictionary is provided, the synonym object dictionary registering a plurality of objects to be regarded as a same object therewith as a synonym object group.

8. The knowledge database according to claim 6,
wherein the objects include any of DNA sequence objects having DNA sequences as object values, protein sequence objects having protein sequences as object values and image objects having images as object values, and any of relations between the DNA sequence objects and the phrase objects, relations between the protein sequence objects and the phrase objects, and relations between the image objects and the phrase objects have relation values previously defined.

9. The knowledge database according to claim 8,
wherein a synonym object dictionary is provided, the synonym object dictionary registering any of a plurality of DNA sequence objects having highly homologous DNA sequences as object values and protein sequence objects having highly homologous protein sequences as object values therewith as a same object group.

10. The knowledge database according to claim 1 or 6,
wherein the phrase objects have phrases in knowledge expressed by a natural language as object values, the knowledge expressed by a natural language being lingualized knowledge, and have values obtained by quantifying frequencies of existence of the respective phrases being close to each other in the lingualized knowledge as relation values of relations between the corresponding phrase objects.

11. The knowledge database according to claim 1 or 6,
wherein the phrase objects have phrases included in an index of a book as object values, and have a value obtained by quantifying a frequency of existence of two phrases being on any of a same page and a same paragraph of the book as a relation value of a relation between the corresponding two phrase objects.

12. The knowledge database according to claim 1 or 6,
wherein the relations between the phrase objects have, as relation values, values obtained by quantifying similarities of patterns in existence of the phrases in knowledge units expressed by a natural language, the similarities of patterns in existence being existing profiles.

13. A method for displaying objects and relations, comprising the steps of:
extracting objects related to an object corresponding to one or a plurality of keywords from a knowledge database based on relations between the objects, the object corresponding to the keywords being a query object, and the knowledge database including the objects having phrases including terms for use in a medical field and/or a biological field as object values and the relations between the objects, the relations having values expressing the relations between the objects quantitatively as relation values; and
displaying the query object and the extracted objects together with the relations therebetween.

14. The method according to claim 13,
wherein the query object is selected by inputting or searching the object values, and the selected query object, the extracted objects and the relations therebetween are displayed on a list or displayed graphically.

15. The method according to claim 13,
wherein objects having strong relations with the query object are obtained hierarchically, and the query object, the hierarchically obtained objects and relations therebetween are displayed on a list or displayed graphically.

16. A method for constructing a knowledge database, comprising the steps of:
extracting phrases used in expression of knowledge of a medical field and/or a biological field from the knowledge expressed by a natural language, the knowledge expressed by the natural language being lingualized knowledge;
generating objects having the phrases as object values;
generating relations between the objects, the relations having values expressing the relations between the objects quantitatively as relations values; and
accumulating the generated objects and the relations between the objects.

17. The method for constructing a knowledge database according to claim 16,
wherein the lingualized knowledge is decomposed into sentences by previously prepared sentence-separating letter strings, each being composed of one letter or a plurality of letters, the sentences are decomposed into phrases by previously prepared phrase-separating letter strings, each being composed of one letter or a plurality of letters, and the phrases obtained are set as the object values, and a frequency of existence of two phrases being close to each other in the lingualized knowledge is set as a relation value of a relation between the corresponding two phrase objects.

18. The method for constructing a knowledge database according to claim 16 or 17,
wherein an index of a book, a table of contents of a book, a title of an academic treatise, a body text of a book, a body text of an academic treatise and/or a body text of a Web page are used as the lingualized knowledge.

19. The method for constructing a knowledge database according to claim 16,
wherein phrases included in the index of the book are used as master data of phrase objects, and a frequency of existence of the two phrases being on a same page of the book is set as a relation value of a relation between the corresponding two objects.

20. The method for constructing a knowledge database according to claim 16,
wherein previously designated optional phrases are used as master data of phrase objects, and a frequency of existence of the two phrases being closer to each other in the lingualized knowledge is set as a relation value of a relation between the corresponding two objects.

21. The method for constructing a knowledge database according to claim 16,
wherein academic treatises are used as the lingualized knowledge, information including titles and/or body texts of the academic treatises is periodically acquired through a network, phrase objects and relations are extracted from the acquired information, and the knowledge database is updated.

22. The method for constructing a knowledge database according to claim 16,
wherein a plurality of objects to be regarded as a same object and relations related thereto are merged.

23. A method for constructing a knowledge database, comprising the steps of:
extracting DNA sequences from information including DNA sequences;
generating DNA sequence objects having the DNA sequence as object values;
generating a relation between two DNA sequence objects, the relation having, as a relation value, a quantitative value obtained by any of homology calculation and experimental measurement for a relation between the corresponding two DNA sequence; and
accumulating the generated DNA sequence objects and the generated relation between the DNA sequence objects.

24. The method for constructing a knowledge database according to claim 23,
wherein phrases included in natural language information related to the DNA sequences are extracted, objects having the extracted phrases as the object values are generated, and relations having defined relation values with the DNA sequence objects corresponding to phrase objects are set.

25. The method for constructing a knowledge database according to claim 24,
wherein information including the DNA sequences and natural language information related thereto is periodically acquired through a network, the DNA sequence objects, the phrase objects and the relations are extracted from the acquired information, and the knowledge database is updated.

26. The method for constructing a knowledge database according to claim 23,
wherein a plurality of the DNA sequence objects having highly homologous DNA sequences as object values are regarded as a same object, and the plurality of DNA sequence objects regarded as the same object and relations related thereto are merged.

27. A method for generating a knowledge database, comprising the steps of:
performing an operation between first and second databases, each database accumulating a plurality of objects having, as object values, phrases expressed by a natural language, and relation values between the plurality of objects, the relations having, as relation values, values expressing relations between the objects quantitatively; and
generating a third knowledge database.

28. A method for displaying a graph, comprising the step of:
displaying a graph for comparing differences between relation values of focused relations in the first and second knowledge databases by use of the first and second databases, each database accumulating a plurality of objects having, as object values, phrases expressed by a natural language, and relation values between the plurality of objects, the relations having, as relation values, values expressing relations between the objects quantitatively.

29. The method according to claim 27 or 28,
wherein the relation values of the relations between the phrase objects included in each knowledge database are standardized between the plurality of knowledge databases.
